# EUROPEAN PATENT APPLICATION

(11) **EP 1 498 116 A1**
(43) Date of publication of application: **19.01.2005**
(21) Application number: 02765521.6
(22) Date of filing: 12.09.2002
(51) Int. Cl.: A61K 9/14, A61K 47/38, A61K 47/02, A61K 47/34

(54) **METHOD OF MANUFACTURING CHEMICAL-CONTAINING COMPOSITE PARTICLES**

(30) Priority: 20.03.2002 JP 2002079400; 20.03.2002 JP 2002079424
(71) Applicant: HOSOKAWA MICRON CORPORATION, Osaka-Shi, Osaka 541-0048 (JP)
(72) Inventor: KAWASHIMA, Yoshiaki, Gifu-shi, Gifu 502-0858 (JP); HOSOKAWA, Masuo, Toyonaka-shi, Osaka 560-0013 (JP); TAKEUCHI, Hirofumi, Gifu-shi, Gifu 502-0006 (JP); YAMAMOTO, Hiromichi, Gifu-shi, Gifu 502-0814 (JP); IWATO, Masaru, Kyoto-shi, Kyoto 601-1371 (JP); SUHARA, Kazuki, Shimogyo-ku, Kyoto-shi, Kyoto 600-8436 (JP); YOKOYAMA, Toyokazu, Kuse-gun, Kyoto 613-0034 (JP); TSUJIMOTO, Hiroyuki, Hirakata-shi, Osaka 573-1127 (JP); KONDO, Akira, Hirakata-shi, Osaka 573-1105 (JP); YOSHITOMI, Tsunehiro, Takatsuki-shi, Osaka 569-0823 (JP); INOUE, Yoshiyuki, Kyoto-shi, Kyoto 601-8173 (JP); EITOKU, Hisato, Hirakata-shi, Osaka 573-1107 (JP); KINOSHITA, Naotoshi, Kyoto-shi, Kyoto 612-8112 (JP)
(74) Representative: Bentz, Jean-Paul
(86) International application number: PCT/JP2002/009377
(87) International publication number: WO 2003/077886

(57) **Abstract**

A strong pressure and a strong shearing force are exerted to a mixture, constituted of two kinds or more of powder materials including a drug powder, while causing the mixture to pass between a press section (26) of a press head (24) and a receiving surface (25) of a cylindrical rotator (23), thereby combining the drug powder with the other powder material. For example, at least one of a drug and a biocompatible polymer is made into a nano particle whose average particle diameter is less than 1000 nm, and the nano particle is made into a composite in accordance with a dry mechanical particle combining method, so as to form a polymer nano composite particle. Thus, it is possible to produce a composite particle, which contains a drug under a stable condition, in a short time, and it is possible to remarkably improve its handling property without losing advantages of the nano particle. As a result, it is possible to favorably apply the foregoing technique to DDS of a powdery drug taken into the body through the lung or a similar drug.

## Description

### TECHNICAL FIELD

The present invention relates to a method for producing a drug containing composite particle which method is effective in modifying a surface of a powder material and in giving a higher performance to the drug containing composite particle.

### BACKGROUND ART

Medical drugs are required to have various properties such as tractability (handling property) in production, masking of bitterness, solubility control, DDS (Drug Delivery System), and the like. Thus, a plurality of materials have been mixed as a composite in order to give necessary properties. Examples of the composite constituted of the plural materials include: a composite constituted of a diluting agent and a drug; a composite obtained by coating a surface of a drug with a lubricant or a coating agent; and the like. Here, the diluting agent improves the handling property of the drug and makes it easier to make the drug into a medical drug. The lubricant lubricates a surface of the drug. The coating agent covers the surface of the drug so as to mask the bitterness of the drug for example.

As a method of combining materials with each other so as to produce the composite, for example, Japanese Unexamined Patent Publication No. 128774/2000 (Tokukai 2000-128774) discloses a method in which: a solution of a binding agent is poured to a mixture obtained by combining a diluting agent powder having a solvent-retaining property with a drug powder, and thus obtained resultant is subjected to high-speed rotational granulation, so as to produce spherical micro particles.

However, the combining method disclosed in the aforementioned publication is a wet-combining method using a solution of a binding agent as a binder. Thus, it is necessary to dry the binder after combining the materials, and it is necessary to adjust temperature in a device used to combine the materials with each other. Further, there is such a problem that: a device used in the wet-combining method generally has a large size, so that it takes a long time to adjust a condition of the device.

Further, a medicinal property of a medicinal drug is deteriorated in terms of its stability when the medical drug is dissolved in liquid compared with a condition under which the medical drug is in a solid phase. Thus, according to the wet-combining method, the medical drug is dissolved in the binder during the combining treatment, so that its stability drops. This condition causes storage stability of the medical drug to drop.

Therefore, in order to combine a material whose medicinal property particularly requires the stability, it is desired to adopt such a combining method that it takes less time to combine materials and the stability of the medicinal property does not drop.

The present invention was devised in order to solve the foregoing problem, and its object is to provide a method for producing a drug containing composite particle which method is effective in modifying a surface of a powder material and in giving a higher handling property to the drug containing composite particle.

### DISCLOSURE OF INVENTION

In order to solve the foregoing problem, a method of the present invention for producing a drug containing composite particle is characterized by including the step of giving a pressure and a shearing force to a mixture, constituted of two or more kinds of powder materials including a drug powder, so as to combine the powder materials with each other.

According to the arrangement, it is possible to combine two or more kinds of powder materials, including a drug powder, with each other without losing the stability of the drug powder, so that it is possible to produce a drug containing composite particle having a higher function such as higher tractability.

Conventionally, a liquid was used as a binder for binding particles so as to combine the powder materials including the drug powder. Thus, it took a long time to adjust a machine or to dry the binder. Further, there was also such a problem that: the drug was dissolved in the binder at the time of the combining operation, so that the stability of the drug dropped.

In contrast, the present invention provides a method in which a pressure and a shearing force are given to a mixture, constituted of two or more kinds of powder materials including a drug powder, so as to make the mixture into a composite. That is, by giving the pressure and the shearing force to the mixture, it is possible to combine the drug powder with the other powder material without using the binder.

Thus, it is not necessary to carry out a preparation step in which temperature in a device is adjusted in advance before combining the powder materials with each other. Further, it is not necessary to carry out a step of drying the binder. As a result, it is possible to combine the powder materials in a shorter time than in a conventional combining operation using the binder. Further, in the combining operation, the powder materials are combined with each other, while keeping its solid state highly stable, without any drop in the stability which is caused by dissolution of the drug powder in the binder.

Further, according to the conventional combining method using the binder, it is impossible to combine materials each of which is degenerated by dissolution in the liquid. However, the method of the present invention for producing a drug containing composite particle is a method which requires no binder. Thus, it is possible to combine the materials, each of which is degenerated by dissolution in the liquid, while keeping the stability thereof. That is, it is possible to select a drug powder and other powder material, which can be used in the combining operation, from wider alternatives than those in the conventional method. As a result, it is possible to produce more kinds of the drug containing composite particles.

Thus, it is possible to combine two or more kinds of powder materials including a drug powder in a short time without dropping the stability of the drug powder, thereby producing the drug containing composite particle. Note that, in the present invention, the combining operation is an operation in which mechanical energy such as a pressure and a shearing force is given to a plurality of powder materials different from each other so as to bond a material to a surface of a specific powder material so that they are integrated. According to the combining method based on the mechanical energy, the powder materials do not react with each other, and a function of other powder material is given to a specific powder material, so that it is possible to obtain a highly functional composite particle.

Further, the mixture may include a diluting agent. Thus, by combining the drug powder with the diluting agent, it is possible to obtain a drug containing composite particle which is favorable in terms of the handling property in producing a medical drug.

In the method of the present invention for producing the drug containing composite particle, it is preferable that the diluting agent is selected from a group of celluloses and starches.

In the method of the present invention for producing the drug containing composite particle, it is preferable that an average particle diameter of the diluting agent powder is not less than one and not more than 10000 times as large as an average particle diameter of the drug powder. Thus, it is possible to more surely combine the diluting agent with the drug powder, so that it is possible to obtain the diluting agent powder whose surface is covered with an even layer of the drug powder due to the combining operation.

In the method of the present invention for producing the drug containing composite particle, it is preferable that an average particle diameter of the diluting agent powder is 1 µm or more and 5000 µm or less.

In the method of the present invention for producing the drug containing composite particle, it is preferable that an average particle diameter of the drug powder is 0.01 µm or more and 500 µm or less.

In the method of the present invention for producing the drug containing composite particle, it is preferable that a ratio at which the drug powder is contained in the drug containing composite particle is 0.01 wt % or more and 90 wt % or less.

In the method of the present invention for producing the drug containing composite particle, it may be so arranged that the drug powder is an antipyretic analgesic or an antiphlogistic.

Further, in order to solve the foregoing problem, a method of the present invention for producing a drug containing composite particle containing a drug and a biocompatible polymer is characterized by including the steps of: making at least one of the drug and the biocompatible polymer into a nano particle whose average particle diameter is less than 1000 nm; and making a mixture containing the nano particle into a composite particle in accordance with a fluid bed dry granulation method or a dry mechanical particle combining method, so as to form a polymer nano composite particle.

According to the method, a particle mixture containing at least one of the drug nano particle and the biocompatible nano particle is made into a composite in accordance with the fluid bed dry granulation method or the dry mechanical particle combining method. Thus, it is possible to obtain a functional micron particle having a nano structure. Therefore, for example, it is possible to produce a drug containing composite particle which enables the drug nano particles to be sufficiently dispersed at the time of use thereof, and it is possible to produce a drug containing composite particle whose drug delivery property is improved by modifying a particle surface of the drug with the biocompatible polymer nano particles. As a result, it is possible to use the production method to produce the medical drug whose handling property is improved without losing advantages of the nano particle.

In order to solve the foregoing problem, a method of the present invention 'for producing a drug containing composite particle is characterized by including: a primary particle formation step of forming primary particles each of which includes nano particles whose average particle diameter is less than 1000 nm; and a combining step of combining the primary particles with each other so that the primary particles are reversibly collected, wherein a drug powder is used as the nano particles or the primary particles.

According to the method, the primary particles each of which includes nano particles are combined with each other so that they can be dispersed and collected, so that a condition under which the primary particles are clumped is controlled. Thus, it is possible to design the drug containing composite particle so that the obtained drug containing composite particle (nano composite particle) is broken into the primary particles each of which includes nano particles and the primary particles are dispersed at the time of use thereof.

Thus, the average particle diameter of the drug containing composite particle is larger than a nano order before being used, so that a volume of the drug containing composite particles is not so large and fluidity thereof are favorable. Also, during (after) use thereof, the drug containing composite particle can be broken into the primary particles so that it is possible to exhibit a function of nano particles. Therefore, it is possible to improve the handling property of the drug containing composite particle without losing advantages of the nano particle.

It is preferable to arrange the method according to the present invention for producing the drug containing composite particle so that each of the primary particles is a nano particle clump obtained by clumping a plurality of the nano particles. When a clumping condition of the nano particle clump is controlled as required, it is possible to break each of the nano particle clumps, obtained as a primary particle by breaking the drug containing composite particle, into nano particles. Therefore, it is possible to use the drug containing composite particle having a large diameter as the nano particles at the time of use thereof. As a result, it is possible to improve the handling property of the drug containing composite particle while sufficiently making use of advantages of the nano particle.

It is preferable to arrange the method according to the present invention for producing the drug containing composite particle so that the nano particle is used as a drug powder. Thus, it is possible to favorably apply the production method of the present invention to production and the like of DDS medical drugs.

It is preferable to arrange the method according to the present invention for producing the drug containing composite particle so as to further include a nano particle formation step of forming the nano particles in accordance with spherical crystallization. In accordance with the spherical crystallization, it is possible to carry out the crystallization and the granulation at the same time, so that it is possible not only to form high quality nano particles but also to improve a condition under which the nano particle is designed.

It is preferable to arrange the method according to the present invention for producing the drug containing composite particle so that: in the combining step, the nano particle clumps are subjected to secondary granulation in accordance with a fluid bed dry granulation method.

In accordance with the fluid bed dry granulation method, the primary particles are dried while spraying liquid containing a binder to the primary particles in a fluid state, so as to secondarily clump the primary particles via the binder. Therefore, it is possible not only to produce the drug containing composite particle with high efficiency and high quality but also to control a condition under which the nano particles are clumped. As a result, it is possible to further improve the handling property of the drug containing composite particle without losing advantages of the nano particle.

In the fluid bed dry granulation method, it is preferable that an average particle diameter of the primary particles is within a range of from 0.01 µm or more to 500 µm or less. Thus, it is possible to efficiently and surely produce the drug containing composite particle obtained by secondarily granulating the primary particles. Note that, in case where the drug containing composite particle is used as a drug taken into the body through the lung (described later), it is preferable that the average particle diameter of the primary particles is within a range of from 0.01 µm or more to 15 µm or less.

Further, in the fluid bed dry granulation method, it is preferable to use a binder for binding the primary particles to each other. An example of the binder is a biocompatible polymer aqueous solution. Thus, it is possible to control a secondary granulation condition of the drug containing composite particle obtained by secondarily granulating the primary particles. Particularly, in case of using a drug as the nano particle, it is preferable to use the biocompatible polymer.

The method according to the present invention for producing the drug containing composite particle may be arranged so that: in the combining step, the primary particles are made to adhere to a surface of each of carrier particles, which are larger than the primary particles in terms of an external diameter, in accordance with a dry mechanical particle combining method.

In the dry mechanical particle combining method, a pressure and a shearing force are given to a particle mixture obtained by mixing the primary particles with the carrier particle, so that a plurality of nano particle clumps are made to adhere to a surface of the carrier particle. Therefore, also by using this method, it is possible not only to produce the drug containing composite particle with high efficiency and high quality but also to control a condition under which the nano particles are clumped. As a result, it is possible to further improve the handling property of the drug containing composite particle without losing advantages of the nano particle.

In the dry mechanical particle combining method, it is preferable that: an average particle diameter of the primary particles is within a range of from 0.01 µm or more to 500 µm or less, and an average particle diameter of the carrier particles is within a range of from 1 µm or more to 500 µm or less. Thus, it is possible to efficiently and surely produce the drug containing composite particle obtained by combining the carrier particle with the primary particle. Note that, in case of using the drug containing composite particle as a drug taken into the body through the lung (described later), it is preferable that the average particle diameter of the primary particles is within a range of from 0.01 µm or more to 15 µm or less, and the average particle diameter of the carrier particles is within a range of from 10 µm or more to 100 µm or less.

In the dry mechanical particle combining method, it is preferable that: a polysaccharide powder or a hydrophilic polymer powder is used as the carrier particle. Thus, it is possible to favorably control a combining condition of the drug containing composite particle obtained by combining the carrier particle with the nano particle clumps, that is, it is possible to favorably control a condition under which the nano particle clumps adhere to a surface of the carrier particle. Particularly, in case of using a drug as the nano particle, it is preferable to use a polysaccharide powder constituted of the celluloses, the starches, and the like, or a hydrophilic polymer powder constituted of polymethyl methacrylate and the like.

It is preferable that the method according to the present invention for producing the drug containing composite particle further includes a carrier particle surface modification step of modifying the surface of the carrier particle, in accordance with a fluid bed dry granulation method or the dry mechanical particle combining method, before carrying out the combining step. The surface modification of the carrier particle in the carrier particle surface modification step may be carried out by smoothing the surface of the carrier particle in accordance with the foregoing techniques, or may be carried out by combining the carrier particle with the lubricant powder in accordance with the foregoing techniques.

In accordance with the carrier particle surface modification step, it is possible to control a condition under which the primary particles adhere to the surface of the carrier particle. Thus, it is possible to control the separability between the primary particle and the carrier particle, so that it is possible to favorably break the drug containing composite particle into the primary particles and to disperse the primary particles.

Use of the method according to the present invention for producing the drug containing composite particle is not particularly limited. The production method is favorably used in a case where it is necessary to sufficiently use properties of the nano particle in using the drug containing composite particle. For example, the production method is so favorably used to produce a powdery drug which is delivered to the lung and taken into the body through the lung. In the present invention, it is possible to favorably control a shape and density of the drug containing composite particle. Thus, in the production of the drug taken into the body through the lung, a predetermined aerodynamic diameter can be designed so that it is possible to optimize an inhalation property of the drug powder.

Further, in order to solve the foregoing problem, a method of the present invention for producing a drug containing composite particle is characterized by including the step of making a mixture, containing nano particles whose average particle diameter is less than 1000 nm and a drug powder whose average particle diameter is larger than the average particle diameter of the nano particles, into a composite particle in accordance with a fluid bed dry granulation method or a dry mechanical particle combining method, so as to modify a surface of the drug powder.

According to the method, the nano particles and the drug particle larger than each of the nano particles are combined with each other in accordance with the fluid bed dry granulation method or the dry mechanical particle combining method. Thus, it is possible to effectively use the nano particles as an agent which modifies the surface of the drug powder, so that it is possible not only to favorably modify the surface of the drug powder but also to favorably control a level of the surface modification compared with the conventional surface modification using the micron particles. Further, the fluid bed dry granulation method and the dry mechanical particle combining method are suitable for mass treatment in the combining operation. Thus, it is possible to further improve the productivity of the drug powder whose surface has been modified.

In the method according to the present invention for producing the drug containing composite particle, it is preferable that a lubricant powder is used as each of the nano particles. It is preferable that a colloidal inorganic compound powder or an interfacial active agent is used as the lubricant powder. Specifically, it is preferable that the colloidal inorganic compound powder is colloidal silica, and it is preferable that the interfacial active agent is magnesium stearate or sugar ester. By using the lubricant powder, it is possible to favorably modify the surface of the drug powder.

Alternatively, the method according to the present invention for producing the drug containing composite particle may be arranged so that a polymer nano particle obtained in accordance with spherical crystallization is used as the lubricant powder. It is particularly preferable that the polymer nano particle is constituted of a lactic acid · glycolic acid copolymer or hydroxymethyl cellulose phthalate. Thus, it is possible not only to favorably modify the surface of the drug powder with a polymer but also to favorably control a level at which the polymer modifies the surface of the drug powder.

In the method according to the present invention for producing the drug containing composite particle, it is preferable that the average particle diameter of the drug powder is within a range of from 0.01 µm or more and 500 µm or less. Thus, it is possible to efficiently and surely produce the drug powder whose surface has been modified with the nano particles, that is, it is possible to efficiently and surely produce the drug containing composite particle according to the present invention.

Use of the method according to the present invention for producing the drug containing composite particle is not particularly limited. The production method is favorably used in a case where it is necessary to sufficiently use properties of the nano particle, e.g., in a case where it is necessary to obtain a great effect from a medical drug or a medical product in using the drug containing composite particle therein. For example, the production method is so favorably used to produce a powdery drug which is delivered to the lung and taken into the body through the lung. In the present invention, it is possible to favorably control a shape and density of the drug containing composite particle. Thus, in the production of the drug taken into the body through the lung, a predetermined aerodynamic diameter can be designed so that it is possible to optimize an inhalation property of the drug powder.

For a fuller understanding of the nature and advantages of the invention, reference should be made to the ensuing detailed description taken in conjunction with the accompanying drawings.

### BRIEF DESCRIPTION OF DRAWINGS

Fig. 1 is a cross sectional view showing an example of a structure of a powder treatment device used to carry out the present invention.
Fig. 2 is a cross sectional view illustrating an operation in giving a pressure and a shearing force to a target material by means of the powder treatment device shown in Fig. 1.
Fig. 3(a) is an image of microcrystalline celluloses that have not been combined.
Fig. 3(b) is an image of ibuprofens that have not been combined.
Fig. 3(c) is an image of a drug containing composite particle of Example 1 which was obtained by combining the microcrystalline cellulose with the ibuprofen.
Fig. 3(d) is a cross sectional image of the drug containing composite particle of Fig. 3(c).
Fig. 4(a) is an image of a potato starch that has not been combined.
Fig. 4(b) is an image of ethenzamides that have not been combined.
Fig. 4(c) is an image of drug containing composite particles of Example 2 which were obtained by combining the potato starches with the ethenzamides.
Fig. 5(a) is an image of corn starches that have not been combined.
Fig. 5(b) is an image of ethenzamides that have not been combined.
Fig. 5(c) is an image of drug containing composite particles of Example 3 which were obtained by combining the corn starches with the ethenzamides.
Fig. 6 is a cross sectional image of the drug containing composite particles of Fig. 5(c).
Fig. 7 is a cross sectional image of a surface portion of each of the drug containing composite particles of Fig. 5(c).
Fig. 8(a) is an image of polymethacrylic acid methyl that has not been combined.
Fig. 8(b) is an image of ethenzamides that have not been combined.
Fig. 8(c) is an image of a drug containing composite particle of Example 4 which was obtained by combining the polymethacrylic acid methyl with a mixture of the ethenzamide and microparticle titanium oxide.
Fig. 9 is a cross sectional image of the drug containing composite particles of Fig. 8(c).
Fig. 10 shows an ultraviolet absorption spectrum of ibuprofen after filtering methanol extract of Example 5.
Fig. 11 shows a measurement result obtained by carrying out X-ray analysis with respect to the microcrystalline cellulose and the ibuprofen that have not been combined.
Fig. 12 shows a measurement result obtained by carrying out X-ray analysis with respect to (i) the drug containing composite particle of Example 5 that was obtained by simply blending the microcrystalline cellulose with the ibuprofen and (ii) the drug-containing composite particle of Example 5 that was obtained by combining the microcrystalline cellulose with the ibuprofen.
Fig. 13 shows a result obtained by evaluating fusing points of the ibuprofen on the basis of differential thermal analysis.
Fig. 14 shows a result obtained by measuring elution ratios of the ibuprofens in water.
Fig. 15 shows an FT-IR spectrum of the drug containing composite particle of Example 6 that was obtained by combining the microcrystalline cellulose with the ethenzamide.
Fig. 16 shows an FT-IR spectrum of the microcrystalline cellulose.
Fig. 17 shows an FT-IR spectrum of the ethenzamide.
Fig. 18 is a schematic showing an example of a structure of a powder treatment device used in a method according to one embodiment of the present invention for producing a drug containing composite particle while illustrating an example where materials are combined with each other.
Fig. 19(a) is a schematic illustrating a granulation process in spherical granulation which is performed as spherical crystallization adopted to a method according to one embodiment of the present invention for producing a drug containing composite particle.
Fig. 19(b) is a schematic showing a granulation process of emulsion solvent diffusion which is performed as spherical crystallization adopted to a method according to one embodiment of the present invention for producing a drug containing composite particle.
Fig. 20 is a schematic showing an example where a drug taken into the body through the lung is used as one utilization embodiment of the present invention.
Fig. 21 is a schematic illustrating a process of clump, granulation, collapse, and dispersion of a drug containing composite particle obtained by a method according to one embodiment of the present invention for producing a drug containing composite particle.
Fig. 22(a) is an image of lactose particles that have not been combined, and shows a specific example of modification of a surface of a carrier particle as one example of the present invention.
Fig. 22(b) is an image of lactose particles having been combined with sugar ester, and shows a specific example of modification of a surface of a carrier particle as one example of the present invention.
Fig. 22(c) is an image of lactose particles having been combined with magnesium stearate, and shows a specific example of modification of a surface of a carrier particle as one example of the present invention.
Fig. 23 is a schematic showing an example of a process according to one embodiment of the present invention for producing a drug containing composite particle.
Fig. 24 is a schematic showing an example where a drug taken into the body through the lung is used as one utilization embodiment of the present invention.
Fig. 25 is a schematic illustrating an example of spherical crystallization adopted to a method according to one embodiment of the present invention for producing a drug containing composite particle, and specifically shows a granulation process of emulsion solvent diffusion.
Fig. 26 is a schematic showing an example of how a dispersion liquid is prepared at a previous stage of a combining step in a method according to one embodiment of the present invention for producing a drug containing composite particle.

### BEST MODE FOR CARRYING OUT THE INVENTION

### [Embodiment 1]

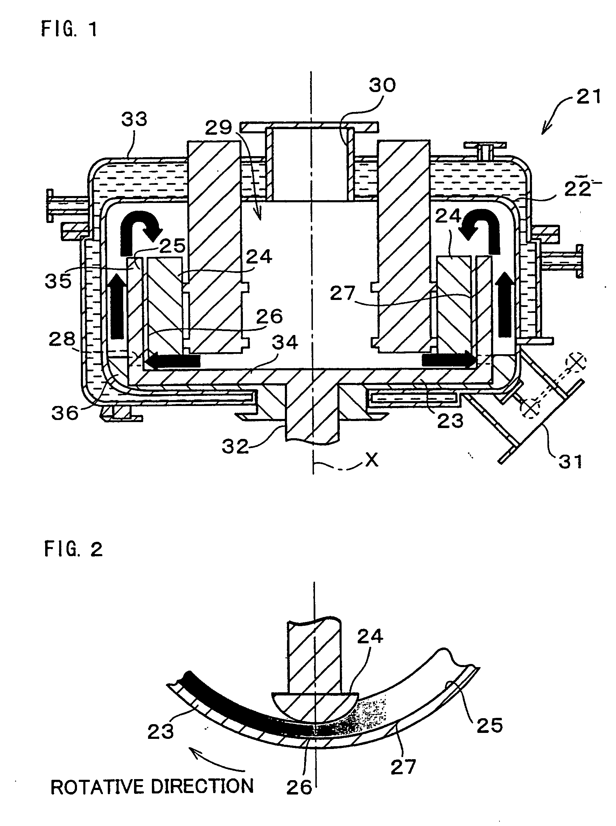

One embodiment of the present invention is described below with reference to Fig. 1 and Fig. 2.

A method of the present embodiment for producing a drug containing composite particle is a method in which: a pressure and a shearing force are given to a mixture constituted of two or more kinds of powder materials including a drug powder so as to combine different kinds of powder with each other, thereby producing the drug containing composite particle.

The drug powder is used as a powder which can be changed into micro particles by receiving a mechanical energy. Further, an average particle diameter of the drug powder is preferably 0.01 µm or more and 10 µm or less, more preferably 0.01 µm or more and 1 µm or less. By using the drug powder whose particle diameter is within the foregoing range, it is possible to surely combine and synthesize the powder with the other powder material on the basis of the combining treatment.

An amount of the drug powder blended is preferably 0.01 wt % or more and 50 wt % or less, more preferably 0.01 wt % or more and 10 wt % or less, with respect to the drug containing composite particle obtained by combining the powder materials.

Examples of the drug powder include: an antipyretic analgesic antiphlogistic; a steroid-type antiphlogistic; an antitumor agent; a coronary vasodilator; a peripheral vasodilating drug; an antibiotic; a synthetic antibacterial agent; an antiviral drug; an antispasmodic agent; a cough suppressant; an expectorant; a bronchodilator; a cardiotonic; a diuretic; a muscle relaxant; a brain metabolic stimulant; a minor tranquilizer; a major tranquilizer; a β-blocker; an antiarrhythmic drug; an antipodagric; a blood coagulation inhibitor; a thrombolytic agent; an antihepatism agent; an antiepileptic; an antihistamine; an antiemetic drug; a hypotensive drug; an antihyperlipemia drug; a sympathomimetic drug; an oral antidiabetic medicine; an oral anticancer agent; an alkaloidal narcotic; a vitamin drop; a pollakiuria therapeutic agent; an angiotensin converting enzyme inhibitor; and the like.

Examples of the antipyretic analgesic antiphlogistic includes: indomethacin; aspirin; sodium diclofenac; ketoprofen; ibuprofen; mefenamic acid; azulene; phenacetin; isopropylantipyrine; acetaminophen; benzadox; butazolidine, phenylbutazone; flufenamic acid; sodium salicylic acid; salicylamide; sasapyrine; etodolac; and the like. Examples of the steroid-type antiphlogistic include: dexamethasone; hydrocortisone; prednisolone; triamcinolone; and the like. Examples of the antitumor agent include: ecabet sodium; enprostil; sulpiride; cetraxate hydrochloride; gefarnate; irsogladine maleate; cimetidine; ranitidine hydrochloride; famotidine; nizatidine; roxatidine acetate hydrochloride; and the like.

Examples of the coronary vasodilator include: nifedipine; isosorbide dinitrate; diltiazem hydrochloride; trapidil; dipyridamole; dilazep hydrochloride; verapamil; nicardipine hydrochloride; verapamil hydrochloride; and the like. Examples of the peripheral vasodilating drug include: ifenprodil tartrate; cinepazide maleate; cyclandelate; cinnarizine; pentoxifylline; and the like. Examples of the antibiotics include: ifenprodil tartrate; cinepazide maleate; cyclandelate; cinnarizine; pentoxifylline; and the like.

Examples of the synthetic antibacterial agent include: nalidixic acid; piromidic acid; pipemidic acid trihydrate; enoxacin; cinoxacin; ofloxacin; norfloxacin; ciprofloxacin hydrochloride; sulfamethoxazole trimethoprim; and the like. Examples of the antiviral drug include: aciclovir; ganciclovir; and the like. Examples of the antispasmodic agent include: propantheline bromide; atropine sulfate; oxapium bromide; timepidium bromide; butylscopolamine bromide; trospium chloride; butropium bromide; N-methylscopolamine methyl hydrogen sulfate; methyloctatropine bromide; and the like.

Examples of the cough suppressant include: tipepidine hibenzate; methylephedrine hydrochloride; codeine phosphate; tranilast; hydrobromic acid dextromethorphan; dimemorfan phosphate; clobutinol hydrochloride; fominoben hydrochloride; benproperine phosphate; eprazinone hydrochloride; clofedanol hydrochloride; ephedrin hydrochloride; noscapine; pentoxyverine citrate; oxeladin citrate; isoaminile citrate; and the like. Examples of the expectorant include: bromhexine hydrochloride; carbocysteine; ethyl cysteine hydrochloride; methyl cysteine hydrochloride; and the like. Examples of the bronchodilator include: theophylline; aminophylline; sodium cromoglycate; procaterol hydrochloride; trimetoquinol hydrochloride; diprophylline; salbutamol sulfate; clorprenaline hydrochloride; formoterol fumarate; orciprenaline sulfate; pirbuterol hydrochloride; hexoprenaline sulfate; bitolterol mesilate; clenbuterol hydrochloride; terbutaline sulfate; mabuterol hydrochloride; fenoterol hydrobromide; methoxyphenamine hydrochloride; and the like.

Examples of the cardiotonic include: dopamine hydrochloride; dobutamine hydrochloride; docarpamine; denopamine; caffeine; digoxin; digitoxin; ubidecarenone; and the like. Examples of the diuretic include: furosemide; acetazolamide; trichlormethiazide; methyclothiazide; hydrochlorothiazide; hydroflumethiazide; ethiazide; cyclopenthiazide; spironolactone; triamterene; florothiazide; piretanide; mefruside; etacrynic acid; azosemide; clofenamide; and the like. Examples of the muscle relaxant include: chlorphenesin carbamate; tolperisone hydrochloride; eperisone hydrochloride; tizanidine hydrochloride; mephenesin; chlorzoxazone; phenprobamate; methocarbamol; chlormezanone; pridinol mesylate; afloqualone; baclofen; dantrolene sodium; and the like.

Examples of the brain metabolic stimulant include: nicergoline; meclofenoxate hydrochloride; taltirelin; and the like. Examples of the minor tranquilizer include: oxazolam; diazepam; clotiazepam; medazepam; temazepam; fludiazepam; meprobamate; nitrazepam; chlordiazepoxide; and the like. Examples of the major tranquilizer include: sulpiride; clocapramine hydrochloride; zotepine; chlorpromazine; haloperidol; and the like.

Examples of the β-blocker include: bisoprolol fumarate; pindolol; propranolol hydrochloride; carteolol hydrochloride; metoprolol tartrate; labetalol hydrochloride; acebutolol hydrochloride; bufetolol hydrochloride; alprenolol hydrochloride; arotinolol hydrochloride; oxprenolol hydrochloride; nadolol; bucumolol hydrochloride; indenolol hydrochloride; timolol maleate; befunolol hydrochloride; bupranolol hydrochloride; and the like. Examples of the antiarrhythmic drug include: procainamide hydrochloride; disopyramide phosphate; cibenzoline succinate; ajmaline; quinidine sulfate; aprindine hydrochloride; propafenone hydrochloride; mexiletine hydrochloride; azimilide hydrochloride; and the like. Examples of the antipodagric include: allopurinol; probenecid; colchicine; sulfinpyrazone; benzbromarone; bucolome; and the like.

Examples of the blood coagulation inhibitor include: ticlopidine hydrochloride; dicumarol; warfarin potassium; (2R, 3R)-3-acetoxy-5-[2-(dimethylamino)ethyl]-2; 3-dihydro-8-methyl-2-(4-methylphenyl)-1; 5-benzodiazepine-4(5H)-one-maleate; and the like. Examples of the thrombolytic agent include: methyl (2E, 3Z)-3-benzylidene-4-(3,5-dimethoxy-α-methylbenzylidene)-N-(4-methylpiperazine-1-yl) succinamate·hydrochloride; and the like. Examples of the antihepatism agent include: (±) r-5-hydroxymethyl-t-7-(3,4-dimethoxyphenyl)-4-oxo-4, 5, 6, 7-tetrahydrobenzo [b] furan-c-6-carboxylic lactone; and the like.

Examples of the antiepileptic include: phenitoin; valproate sodium; metharbital; carbamazepine; and the like. Examples of the antihistamine include: chlorpheniramine maleate; clemastine fumarate; mequitazine; alimemazine tartrate; cychloheptadine hydrochloride; bepotastine besilate; and the like. Examples of the antiemetic drug include: difenidol hydrochloride; metoclopramide; domperidone; betahistine mesilate; trimebutine maleate; and the like.

Examples of the hypotensive drug include: hydrochloride dimethylaminoethyl reserpinic acid; rescinamine; methyldopa; prazosin hydrochloride; bunazosin hydrochloride; clondine hydrochloride; budralazine; urapidil; N-[6-[2-[(5-bromo-2-pyrimidinyl)oxy]ethoxy]-5-(4-methylpheny 1)-4-pyrimidinyl]-4-(2-hydroxy-1,1-dimethylethyl) benzenesulfonamide sodium salt; and the like. Examples of the antihyperlipemia drug include: pravastatin sodium; fluvastatin sodium salt; and the like. Examples of the sympathomimetic drug include: dihydroergotamine mesylate; isoproterenol hydrochloride; etilefrine hydrochloride; and the like.

Examples of the oral antidiabetic agent include: glibenclamide; tolbutamid; glymidine sodium salt; and the like. Examples of the oral anticancer agent include: marimastat; and the like. Examples of the alkaloidal narcotic include: morphia; codeine; cocaine; and the like.

Examples of the vitamin drop include: vitamin B1; vitamin B2; vitamin B6; vitamin B12; vitamin C; folic acid; and the like. Examples of the pollakiuria cure include: flavoxate hydrochloride; oxybutynin chloride; terolidine hydrochloride; and the like. Examples of the angiotensin converting enzyme inhibitor include: imidapril hydrochloride; enalapril maleate; alacepril; delapril hydrochloride; and the like.

The powder material used in the method of the present embodiment for producing the drug containing composite particle is a powder material which can be made into micro particles by exerting mechanical energy thereto.

Examples of powder material other than the drug powder include: a diluting agent; a lubricant; a coating agent; an ultraviolet ray scattering agent, and the like. By using a powder material corresponding to a property required in the drug containing composite particle, it is possible to give a necessary property to the drug containing composite particle.

For example, by using the diluting agent as the powder material, it is possible to improve a compressing property in turning the drug containing composite particle into a medical drug, thereby obtaining an extremely favorable medical drug. Further, by using the lubricant and the coating agent, for example, it is possible to mask the drug bitterness of the drug containing composite particle, and it is possible to suppress the solubility of the drug. Further, by using the ultraviolet ray scattering agent, it is possible to give an ultraviolet preventing function to the drug containing composite particle. Note that, as a powder material other than the drug powder, any powder material may be used as long as the powder material corresponds to a property required in the drug containing composite particle. A single kind of the powder material may be used, or a plurality of kinds thereof may be used.

Examples of the diluting agent include: celluloses such as microcrystalline cellulose, methyl cellulose, carmellose sodium, carmellose calcium, low substituted hydroxypropylcellulose, and the like; starches such as wheat starch, rice starch, corn starch, potato starch, hydroxypropyl starch, carboxymethyl starch sodium, α-cyclodextrin, β-cyclodextrin, and the like; polymers such as polymethyl methacrylate (PMMA), and the like. Among the diluting agents described above as examples, it is preferable to use microcrystalline cellulose, corn starch, potato starch, and polymethyl methacrylate.

Examples of the lubricant include: lactose; saccharose; mannitol; sorbitol; and the like.

Examples of the coating agent include: hydroxypropyl cellulose; polyethylene glycol; lactose; saccharose; hydroxypropyl methyl; calcium carbonate; talc; titanium oxide; gum arabic; crystalline cellulose; carboxymethylethyl cellulose; gelatin; and the like.

Examples of the ultraviolet ray scattering agent include: microparticle titanium oxide; microparticle zinc oxide; and the like.

As the powder treatment device used in carrying out the method of the present embodiment for producing the drug containing composite particle, any device can be used as long as the device can exert a strong pressure and a strong shearing force to the powder materials including the drug powder. However, it is preferable to use a device which can exert such pressure that 3 × 10³ Pa or more and 3 × 10⁷ Pa or less and such a shearing force that 1 × 10³ Pa or more and 1 × 10⁷ Pa. As the powder treatment device, it is possible to use a mixer having a strong stirring force, a kneader, a ball mill, and the like, for example.

The following description will explain an example of the powder treatment device, which can be used to produce the drug containing composite particle of the present invention, with reference to Fig. 1 and Fig. 2.

As shown in Fig. 1, schematically, the powder treatment device 21 includes: a casing 22 which forms a substantially-cylindrical closed space; a cylindrical rotator 23, provided in the casing 22, which has a cylindrical shape with a bottom; and press heads 24, provided in the cylindrical rotator 23, each of which generates a pressure and a shearing force toward an internal face of the cylindrical rotator 23 so as to treat a target material.

The cylindrical rotator 23 is rotated, so that a receiving face 25 formed on an internal face of the cylindrical rotator 23 and the press head 24 are relatively rotated, thereby exerting a pressure and a shearing force to a target material 27 existing in a press section 26 formed as a gap between the receiving face 25 and the press head 24 as shown in Fig. 2. In this manner, the combining treatment is carried out.

As shown in Fig. 1, the casing 22 internally includes the cylindrical rotator 23, having a substantially cylindrical shape, which is rotatable around a rotation axis X extending in a perpendicular direction. The cylindrical rotator 23 includes: a rotation axis 32; a bottom portion 34 connected to the rotation axis 32; and a cylinder side wall 35 connected to the bottom portion 34.

In the powder treatment device 21 used in the present embodiment, slits 28 penetrating the cylinder side wall 35 of the cylindrical rotator 23 are provided on a plural portions around the bottom portion 34 of the cylindrical rotator 23 so as to function as target material discharging means for actively circulating the target material 27 to the press section 26. Each of the slits 28 discharges a part of the target material 27 retained in the press section 26 to the outside of a treatment space 29 while driving the cylindrical rotator 23 to rotate. As described later, all the target materials 27 are sequentially circulated and supplied to the press section 26.

The casing 22 constituting the powder treatment device 21 is supported by a supporting member (not shown), and is fixed on a table. In the casing 22, there is formed a closed space 29 for treating the target material 27. The casing 22 has a target material inlet 30. Further, a target material outlet 31 which allows the target material 27 to be picked up after completing the treatment is provided on a part of a peripheral portion of the bottom of the casing 22. According to this arrangement, it is possible to sequentially treat the target materials 27.

The rotation axis 32 is rotatably provided on the table (not shown) via a roller bearing (not shown). Then, a motor provided on the table and a driving belt (not shown) provided on the motor cause a driving force to be conveyed to a pulley (not shown) of the rotation axis 32, so that the cylindrical rotator 23 is driven to rotate. The cylindrical rotator 23 is driven to rotate, so that a centrifugal force causes the target material 27 to be pressed against the receiving face 25 of the cylindrical rotator 23.

The bottom portion 34 of the cylindrical rotator 23 has (i) a function for connecting the rotation axis 22 to the cylinder side wall 35 of the cylindrical rotator 23 and (ii) a function as retaining means for retaining the target material 27. That is, the bottom portion 34 and the cylinder side wall 35 are in such a relationship that surfaces of them are bent, so that it is possible to avoid such condition that the target material 27 is not sufficiently treated and escapes downwards from the press section 26 when the cylindrical rotator 23 rotates.

An internal face of the cylinder side wall 35 functions as a receiving surface 25 for receiving the target material 27 which tends to move outside due to a centrifugal force. That is, the target material 27 is retained in the press section 26, and cooperation of the receiving surface 25 and the press head 24 gives the target material 27 a pressure and a searing force, thereby carrying out the powder treatment.

In the periphery of a bottom of the cylinder wall section 35, a plurality of slits 28 are formed as shown in Fig. 1. Each of the slits 28 pierces the receiving surface 25, that is, the cylinder wall section 35. The slits 28 are symmetrically provided. For example, two slits 28 are provided in total. Each of the slits 28 discharges a part of the target material 27 retained in the press section 26 to the outside of the press section 26, and functions as means for discharging the target material 27. The slit 28 is formed so that its lower opening area is larger than its upper opening area so as to discharge more target materials 27 positioned lower.

In the present embodiment, the slit 28 is formed in a flume-like shape whose cross-sectional surface is semicircle. The target material 27 is pressed against the receiving surface 25 due to the centrifugal force, and receives a gravity at the same time. Thus, in case of the cylinder wall section 35 shown in Fig. 1, the target material 27 moves downward in a perpendicular direction, so that the target material 27 tends to be deposited on a periphery of a border between the receiving surface 25 and the bottom section 34. The target material 27 deposited there increases a rotational load of the cylindrical rotator 23 and inhibits circulation of the target material 27 into the press section 26. Therefore, the target material 27 deposited there is positively discharged via the slit 28 so as to solve the foregoing disadvantage, thereby improving an efficiency in the powder treatment.

According to the arrangement, most target material 27 existing in the press section 26 is discharged to the outside of the press section 26 via the slit 28. Therefore, the target material 27 is held by the press section 26 for a certain time so that a pressure and a shearing force are exerted to the target material 27, thereby carrying out the powder treatment without fail.

In the cylindrical rotator 23, a press head 24 is provided so as to have a predetermined gap between the press head 24 and the receiving surface 25. The press head 24 gives a pressure and a shearing force to the target material 27 in cooperation with the receiving surface 25. Thus, as shown in Fig. 2, a horizontal cross-sectional surface of the press head 24 is semicircular for example. According to the arrangement, it is possible to consolidate the target material 27 which is likely to enter the gap between the press head 24 and the receiving surface 25, so that it is possible to obtain an advantageous effect in combining and spheroidizing powder particles.

Further, in case of forming the horizontal cross-sectional surface of the press head 24 in a semicircular shape, a curvature of the press head 24 is made higher than a curvature of the receiving surface 25. Thus, the target material 27 fixed on the receiving surface 25 of the cylindrical rotator 23 receives a strong pressure and a strong shearing force while rotation of the cylindrical rotator 23 causes the target material 27 to pass through the press section 26. Here, when a mixture of plural kinds of materials is used as the target material 27, the target material 27 receives a strong pressure and a strong shearing force, so that it is possible to obtain the following advantages: particles are combined with each other; a particle surface is modified; a particle shape is controlled; particles are minutely and accurately mixed in a dispersing manner at a particle level (powder fusion); and a similar advantage. As a result, it is possible to control particle properties.

Further, it may be so arranged that the press head 24 is fixed as in the casing 22, or it may be so arranged that: the press head 24 is driven so as to rotate by means of any driving means so that the press head 24 is positively rotate in opposition to the receiving surface 25. That is, a rotation direction or a rotation speed of the press head 24 is set as required, so that it is possible to more minutely set a relative rotation speed between the press head 24 and the receiving surface 25, thereby setting a most appropriate treatment condition for the target material 27. Note that, it may be so arranged that temperature of the press head 24 is controlled. Although not shown, when a heat medium path is provided in the press head 24, it is easy to set a most appropriate treatment condition depending on a thermal property of the target material 27.

Circulation blades 36 are provided on peripheral lower portions of the casing 22. A plurality of the circulation blades 36 are provided along a peripheral direction of the cylindrical rotator 23, and the number of the circulation blades 36 is an arbitrary. Each of the circulation blades 36 circulates the target material 27, discharged from the slit 28 to the outside of the cylindrical rotator 23, into the press section 26 again. The circulation blade 36 is formed so as to be fitted into an internal shape of the casing 22 so that: the target material 27 is raised up along a peripheral surface of the cylinder side wall 35, and the target material 27 is made to circulate beyond an upper end of the cylinder wide wall 35 into the treatment space 29 of the cylindrical rotator 23, and the target material 27 is smoothly and surely transported so as to be brought back to the press section 26.

The target material 27 is treated by using the powder treatment device 21 arranged in the foregoing manner, so that the target material 27 is pressed against the receiving surface 25 of the cylindrical rotator 23 due to a centrifugal force, which causes the target material 27 to be consolidated, so that a layer of the consolidated target material 27 is generated on the receiving surface 25. While, a part of the consolidated target material 27 is discharged to the outside of the cylindrical rotator 23 via the slit 28, and the target material 27 existing in the cylindrical rotator 23 is stirred by the press head 24 to some extent. Therefore, it is possible to quickly promote the combining treatment of the target material 27.

As described above, according to the powder treatment device 21, the target material 27 is placed in the treatment space 29 of the casing 22 via a target material inlet 30 of the casing 22, and the cylindrical rotator 23 and the press head 24 exert a strong pressure and a strong shearing force to the target material 27, thereby carrying out the combining treatment. Further, the slit 28 is provided on the side wall of the cylindrical rotator 23, so that the mixture is transported via the slit 28 of the cylindrical rotator 23 to the outside of the treatment space 29 of the cylindrical rotator 23. Further, as shown in Fig. 1, the target material 27 transported to the outside is transported to an upper portion of the cylindrical rotator 23 by the circulation blade 36, and is brought back to the inside of the cylindrical rotator 23 again, so that the target material 27 receives a pressure and a shearing force again. In this manner, the target material 27 repeatedly receives the strong pressure and the strong shearing force that are exerted by the cylindrical rotator 23 and the press head 24, so that the combining treatment is effectively carried out.

Note that, the power treatment device 21 may be arranged so as to change an atmosphere of an internal space of the casing 22, that is, an atmosphere of the treatment space 29 depending on kinds of the target material 27. For example, it may be so arranged that various kinds of gas such as inert gas and heating gas are introduced into the casing 22 via the target material inlet 30, or it may be so arranged that a pressurization/vacuum pump or the like is used to pressurize or depressurize the inside of the casing 22. In this case, a sealing member (not shown) is provided between the casing 22 and the rotational axis 32 of the cylindrical rotator 23, so as to surely adjust an atmosphere in the treatment space 29.

Around the casing 22, there is provided a jacket 33 mainly for adjusting temperature of the treatment space 29. To the jacket 33, a heating medium or a coolant is cyclically supplied from an additionally provided tank (not shown). Thus, it is possible to adjust internal temperature of the casing 22. For example, in case where a drug which may be degenerated by temperature variation is treated as the target material 27, the heating medium or the coolant is cyclically supplied to the jacket 33, so that it is possible to set temperature of the target material 27 to be a desired temperature, thereby preventing the drug from degenerating.

Note that, the foregoing description explained the case where the powder treatment device 21 is used to carry out the powder treatment. However, a powder treatment device for carrying out the method of the present embodiment for producing drug containing composite particle is not limited to this. For example, it is possible to carry out the method of the present embodiment for producing a drug containing composite particle by using a mixer, a stirring device, a ball mill, or the like, exerting a strong pressure and a strong shearing force to powder materials, which has strong stirring power.

Next, the following description will explain other condition in case of carrying out the method of the present embodiment for producing the drug containing composite particle. In the present embodiment, a mixture constituted of two or more kinds of powder materials including a drug powder is treated by the powder treatment device as a target material, so that it is possible to obtain a drug containing composite particle in which materials are combined with each other.

How two or more kinds of the powder materials containing the drug powder are placed in the powder treatment device is not particularly limited. For example, it may be so arranged that two or more kinds of powder materials are mixed in advance and then a mixture obtained in this manner is placed in the powder treatment device, or it may be so arranged that the powder materials are separately placed in the powder treatment device and then they are mixed in the powder treatment device. An order in which the powder materials are separately placed in the powder treatment device is not particularly limited, and two or more kinds of powder materials may be simultaneously placed in the powder treatment device.

Note that, in case of placing the powder materials separately in the powder treatment device, it may be so arranged that the same kind or different kinds of powder materials are placed in the powder treatment device in doses and then are combined. The powder materials are placed in the powder treatment device in doses, so that there are formed layers of the powder materials corresponding to the number of times the powder materials are placed therein. That is, a powder material is further added to the drug containing composite particle obtained by combining two or more kinds of powder materials, so that a layer of the powder material is further formed on a surface of the drug containing composite particle. In this manner, the power materials are placed in the powder treatment device in doses, so that it is possible to obtain a drug containing composite particle whose surface has a plurality of layers of powder materials.

Note that, the powder materials placed in the powder treatment device in doses may be the same kind or different kinds. The powder materials of the same kind are placed in the powder treatment device in doses, so that a plurality of layers of the powder material of the same kind are formed. As a result, it is possible to more surely cover a surface of the powder material, which is to be coated, with a powder material, which coats the surface of that powder material. Further, different kinds of powder materials are placed in the powder treatment device in doses so as to correspond to the respective kinds, so that it is possible to obtain a drug containing composite particle in which a plurality of layers of different kinds of power materials are formed on the surface of the powder material which is coated. Thus, it is possible to obtain a drug containing composite particle in which layers of plural kinds of powder materials are formed. For example, a drug powder which functions by dissolving in the stomach is combined with a drug containing composite particle obtained by combining a coated powder material with a drug powder which functions by dissolving in the intestine, so that layers of plural kinds of powder materials are formed. After being taken in the human body, thus obtained drug containing composite particle sequentially exhibits different functions with time. In this manner, it is possible to give a plurality of properties to the drug containing composite particle.

As long as temperature in combining powder materials is within a range which does not cause the powder materials to degenerate, the temperature is not particularly limited. Further, in case of using powder materials degenerated by heat, the powder materials are combined while cooling the powder materials or the powder treatment device in order to prevent the rising temperature in the combining the materials from degenerating the powder materials. Thus, it is possible to prevent the powder materials from degenerating in combining the powder materials.

A time for which the pressure and the shearing force are exerted in combining the powder materials is determined depending on a size of the powder treatment device, kinds of the combined powder materials, amounts of the combined powder materials, and the like, and the time is not particularly limited. However, it is possible to set the treatment time to be 5 to 20 minutes for example. Further, an end of the treatment is determined by evaluating product properties in accordance with a test performed by changing the treatment time (properties of the drug containing composite particle which was obtained by changing the treatment time).

Further, the method of the present embodiment for producing the drug containing composite particle is a method in which a binder is not used, so that it is not necessary to adjust temperature in the powder treatment device to be suitable temperature, and it is not necessary to dry the binder. Thus, it is possible to obtain the drug containing composite particle in a shorter time than in a conventional combining method using the binder.

In order to exert the pressure and the shearing force to the mixture constituted of two or more kinds of power materials including a drug powder, a rotation speed of the powder treatment device is determined depending on a size of the powder treatment device, kinds of the combined powder materials, amounts of the combined powder materials, and the like, but the rotation speed of the powder treatment device is preferably 50 rpm (revolutions per minute) or more and 5000 rpm or less, more preferably 100 rpm or more and 3000 rpm or less.

The drug containing composite particle obtained by the production method of the present embodiment is produced by combining two or more kinds of powder materials including a drug powder. An example of the drug containing composite particle is obtained by combining a core powder material (hereinafter, referred to as a main particle) with a powder material (hereinafter, referred to as sub particles) constituting a layer which covers the core powder material. In the drug containing composite particle, a surface of the main particle is covered with the sub particles, so that it is possible to modify the surface of the main particle.

Note that, in order to cover the surface of the main particle with the sub particles, it is preferable that a shape of the main particle is spherical, and it is preferable that a particle diameter of the main particle and a particle diameter of the sub particle largely different from each other. Further, it is preferable that the particle diameter of the main particle is not less than 10 times and not more than 100 times as large as the particle diameter of the sub particle.

For example, a diluting agent powder which functions as the main particle and a drug powder which functions as the sub particle are combined with each other, and a drug containing composite particle obtained by combining the diluting agent powder with the drug powder is combined with other sub particles, so that it is possible to improve solubility of the drug powder, and it is possible to control fluidity of the drug containing composite particle, and it is possible to give properties suitable for DDS (Drug Delivery System) to the drug containing composite particle.

A specific example of the drug containing composite particles having the properties suitable for DDS is obtained by combining a starch, having a 75 µm particle diameter, which functions as a main particle, with an antiphlogistic powder which functions as a sub particle. When the drug containing composite particle is inhaled, the drug containing composite particle adheres to the throat membrane, so that the antiphlogistic is absorbed through the membrane. Here, fine particles are more likely to fit in the membrane than liquid, so that it is possible to retain the antiphlogistic powder in the membrane for a long time by causing the drug containing composite particles to be inhaled and to adhere to the membrane. Thus, it is possible to deliver the antiphlogistic to the membrane with high efficiency, and it is possible to exhibit medical effects for a long time. Note that, a starch is used as the main particle, so that the drug containing composite particle is safe even when it is taken into the body without adhering to the membrane.

Note that, when a particle diameter of the drug containing composite particle is set to be 1 µm to 5 µm, it is possible to deliver the drug containing composite particle to the lung.

Further, a specific example of the drug containing composite particle using plural kinds of drug powder as the sub particles is obtained by forming a layer of a drug powder which functions by dissolving in the intestine and a layer of drug powder which functions by dissolving in the stomach in this order from the main particle side. The drug containing composite particle causes appropriate drugs to effectively function in the stomach and the intestine respectively.

Further, the drug containing composite particle may be arranged so that a surface thereof is covered with at least one of a lubricant and a coating agent (protective agent). Thus, it is possible to mask the bitterness of the drug powder, and it is possible to suppress the solubility.

As described above, two or more kinds of powder materials including a drug powder are combined in the foregoing manner, so that it is possible to obtain a drug containing composite particle in which: a masking property with respect to drug bitterness, a handling property of the drug, a compressing property, solubility, a DDS property, and the like are adjusted.

### [Example 1]

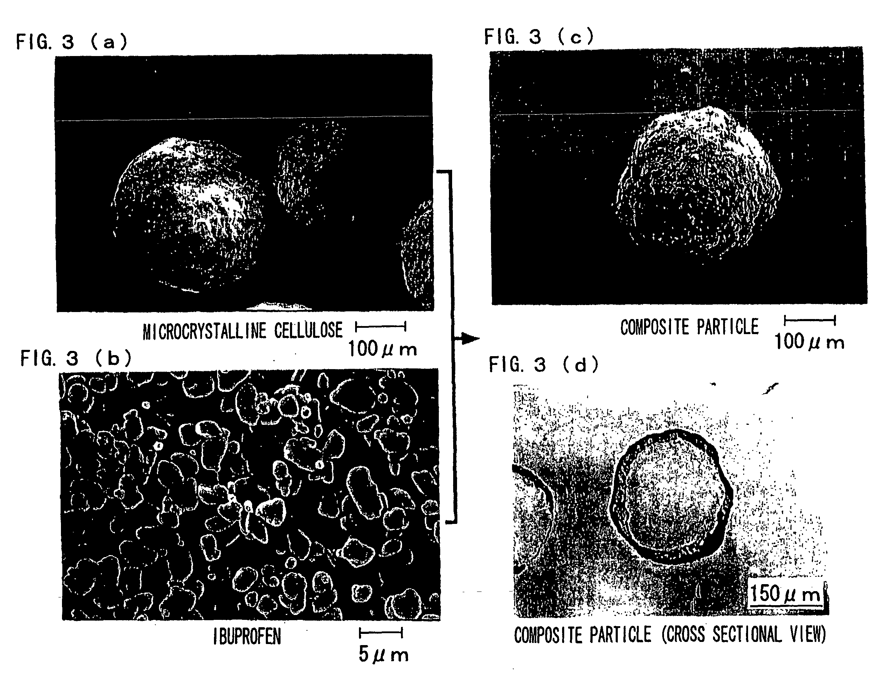

The following description will explain one Example of the present invention with reference to Fig. 3. In the present example, 40 g of microcrystalline cellulose whose average particle diameter was 290 µm and 16 g of ibuprofen (drug powder) whose average particle diameter was 27 µm were used as powder materials, and these powder materials were combined by the powder treatment device, thereby preparing drug containing composite particles. Note that, ibuprofen is an antipyretic analgesic.

The preparation of the drug containing composite particles was carried out under such condition that: treatment temperature was 26°C, and the number of revolutions of the cylindrical rotator 3 was 1300 rpm, and a total time of the treatment was 20 minutes. In the present example, an operation in which 4 g of ibuprofen was placed in the powder treatment device and the combining treatment was carried out for 5 minutes was repeated four times, thereby carrying out the combining treatment for 20 minutes in total. That is, 4 g of ibuprofen was placed on 40 g of microcrystalline cellulose in four doses, and the 5-minute combining treatment was carried out each time ibuprofen was placed. The combining treatment was carried out for 20 minutes in total, thereby preparing the drug containing composite particles. Note that, in the present example, a mechanofusion (registered trademark: product of Hosokawa Micron Corporation) system having a structure described with reference to Fig. 1 was used.

Fig. 3(a) shows an image (150 power) of microcrystalline cellulose that has not been combined, and Fig. 3(b) shows an image (2500 power) of ibuprofen that has not been combined, and Fig. 3(c) shows an image (150 power) of the drug containing composite particle obtained by combining microcrystalline cellulose with ibuprofen, and Fig. 3(d) shows a cross sectional image (150 power) of the drug containing composite particle. Note that, the "power" of each image is a magnifying power of an electronic microscope in imaging the foregoing materials. Further, in case of describing a magnifying power of an image in Examples explained later, the magnifying power in imaging the foregoing materials is used as in the present example.

As shown in Fig. 3(c) and Fig. 3(d), the microcrystalline cellulose and the ibuprofen are combined with each other, so that it is possible to obtain the drug containing composite particle in which a surface of microcrystalline cellulose is entirely covered with ibuprofen.

### [Example 2]

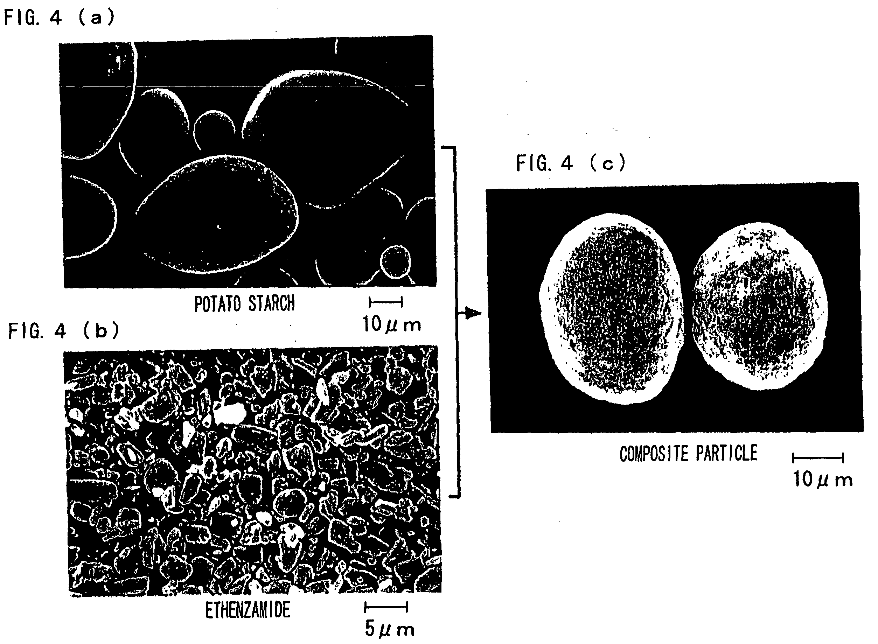

The following description will explain another example of the present invention with reference to Fig. 4. The same operation as in Example 1 was carried out except that: 40 g of potato starch whose average particle diameter was 35 µm and 16 g of ethenzamide (drug powder) whose average particle diameter was 3 µm were used as powder materials, and the treatment temperature was 29°C, and the number of revolutions of the cylindrical rotator 3 was 2950 rpm, thereby preparing a drug containing composite particle. Also in the present example, 4 g of ethenzamide was placed in the powder treatment device in four doses as in Example 1. Note that, ethenzamide is an antiphlogistic.

Fig. 4(a) shows an image (1000 power) of potato starch that has not been combined, and Fig. 4(b) shows an image (2500 power) of ethenzamide that has not been combined, and Fig. 4(c) shows an image (1500 power) of the drug containing composite particle obtained by combining potato starch with ethenzamide.

As shown in Fig. 4(c), the potato starch and the ethenzamide are combined with each other, so that it is possible to obtain the drug containing composite particle in which a surface of potato starch is entirely covered with ethenzamide.

### [Example 3]

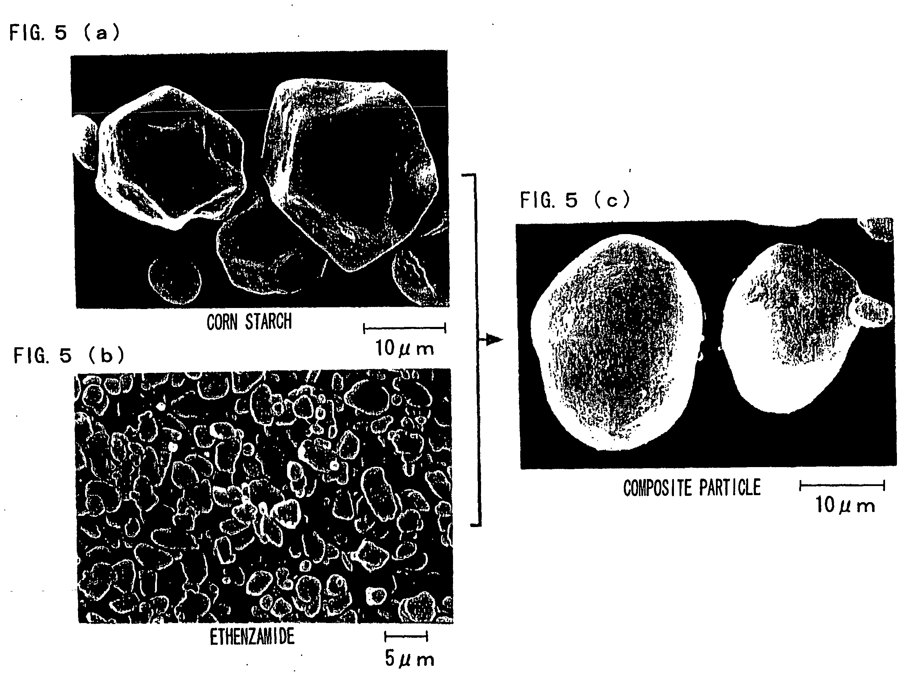

The following description will explain another example of the present invention with reference to Fig. 5 to Fig. 7. The same operation as in Example 1 was carried out except that: 35 g of corn starch whose average particle diameter was 20 µm and 14 g of a mixture obtained by mixing ethenzamide whose average particle diameter was 3 µm with microparticle titanium oxide whose average particle diameter was 0.015 µm so that a weight ratio thereof was 1 : 1 were used as powder materials, and the treatment temperature was 29°C, and the number of revolutions of the cylindrical rotator 3 was 3500 rpm, thereby preparing a drug containing composite particle. Also in the present example, 3.5 g of the mixture was placed in the powder treatment device in four doses as in Example 1. Further, the mixture obtained by mixing ethenzamide with microparticle titanium oxide was used to examine a structure of a film of drug powder in case where the drug powder was placed in the powder treatment device in doses.

Fig. 5(a) shows an image (2500 power) of corn starch that has not been combined, and Fig. 5(b) shows an image (2500 power) of ethenzamide that has not been combined, and Fig. 5(c) shows an image (2500 power) of the drug containing composite particle obtained by combining corn starch with the mixture obtained by mixing ethenzamide with microparticle titanium oxide.

As shown in Fig. 5(c), there is formed the drug containing composite particle in which a surface of corn starch is entirely covered with the mixture by the combining treatment.

Further, comparison between Fig. 5(a) and Fig. 5(c) shows that protruding portions of corn starch are removed so that its shape is substantially spherical by the combining treatment. That is, the comparison shows that the combining treatment causes corn starch to have a layer of the mixture on its surface and to have a spherical shape.

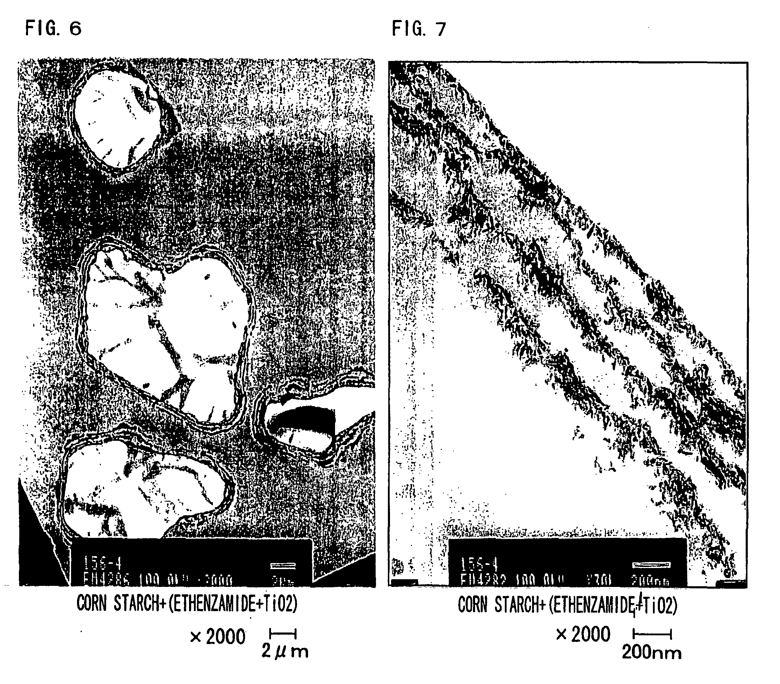

Fig. 6 shows an image (2000 power) of the drug containing composite particle obtained by combining corn starch with the mixture. Further, Fig. 7 shows a cross sectional image (3000 power) taken in a periphery of a surface of the drug containing composite particle shown in Fig. 6. Each of Fig. 6 and Fig. 7 shows that four layers are formed on the surface of corn starch so as to correspond to four doses in placing the mixture in the powder treatment device. That is, the powder material is placed in the powder treatment device in doses at the time of combining, so that layers corresponding to the number of doses are formed. This shows that: different kinds of powder materials are sequentially placed in the powder treatment device, so that there are formed layers of plural powder materials which are different from each other in terms of a kind.

### [Example 4]

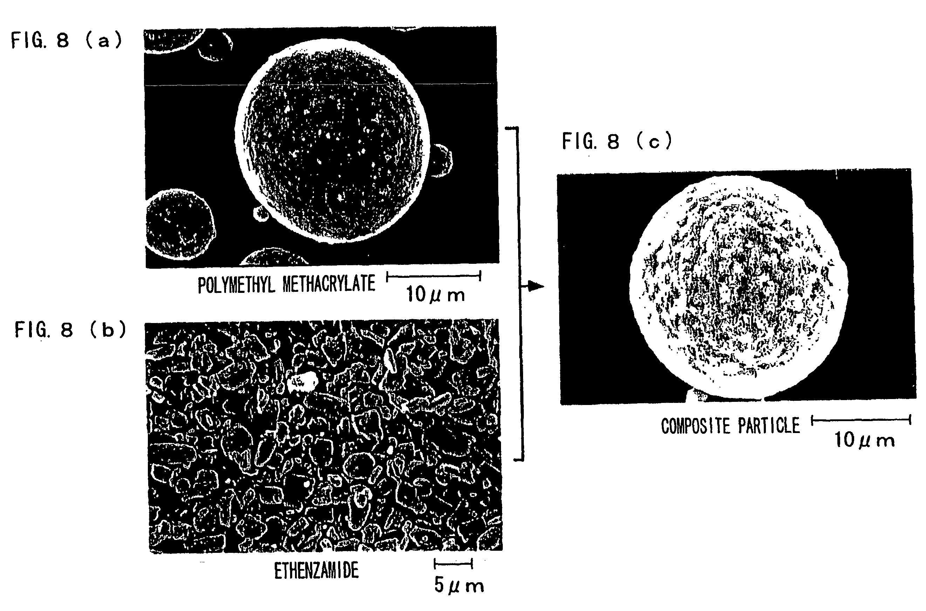

The following description will explain another example of the present invention with reference to Fig. 8 to Fig. 9. 30 g of polymethyl methacrylate (PMMA) whose average particle diameter was 10 µm and a mixture obtained by mixing 6 g of ethenzamide (drug powder) whose average particle diameter was 3 µm with 6 g of microparticle titanium oxide whose average particle diameter was 0.015 µm were used as powder materials, so as to carry out the combining treatment under such condition that: the treatment temperature was 33°C, and the number of revolutions of the cylindrical rotator 3 was 4800 rpm, and the mixture was placed in the powder treatment device at once, and the treatment time was 20 minutes. Note that, the powder treatment device used in the combining treatment was the same powder treatment device as in Example 1.

Fig. 8(a) shows an image (3000 power) of polymethyl methacrylate that has not been combined, and Fig. 8(b) shows an image (2500 power) of ethenzamide that has not been combined, and Fig. 8(c) shows an image (3000 power) of the drug containing composite particle obtained by combining polymethyl methacrylate with the mixture obtained by mixing ethenzamide with microparticle titanium oxide.

As shown in Fig. 8(c), there is formed the drug containing composite particle in which a surface of polymethyl methacrylate is entirely covered with the mixture by the combining treatment.

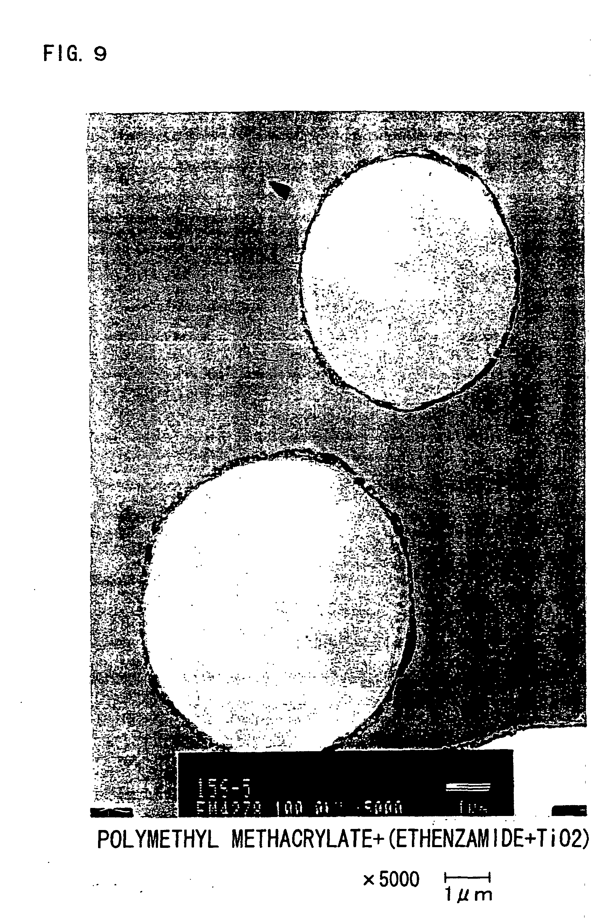

Fig. 9 shows a cross sectional image (5000 power) of the drug containing composite particle of the present example. As shown in Fig. 9, a surface of polymethyl methacrylate has a layer, having an even thickness, which is constituted of the mixture obtained by mixing the ethenzamide with the microparticle titanium oxide.

### [Example 5]

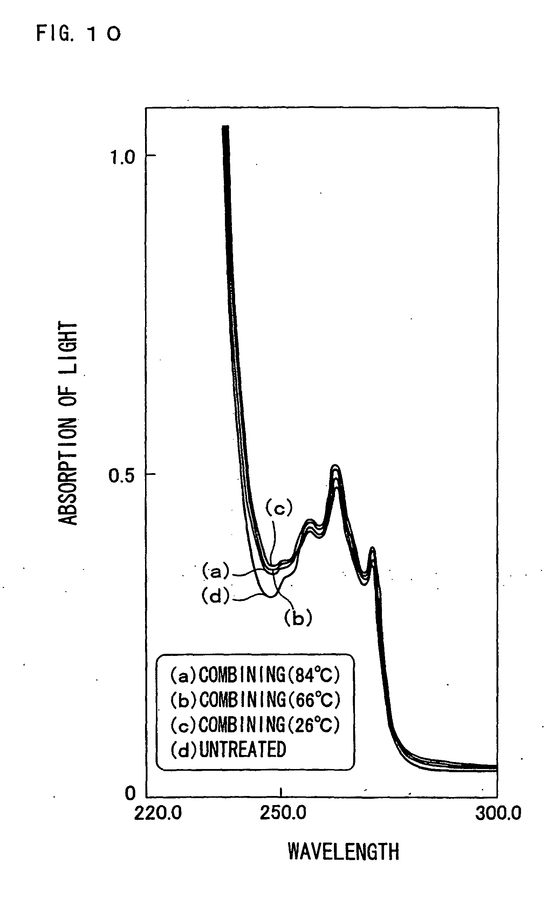

The following description will explain another example of the present invention with reference to Fig. 10 to Fig. 14. In order that a drug containing composite particle obtained by combining two or more kinds of powder materials including a drug powder can function as a drug, it is necessary that the combining treatment does not chemically change the drug, that is, it is necessary that the combining treatment does not cause the drug to degenerate. Then, ultraviolet absorption spectrum (UV spectrum) analysis, X-ray analysis, and measurement of fusing points were carried out with respect to a drug containing composite particle before and after the combining treatment, so as to examine how the combining treatment influences the drug.

40 g of microcrystalline cellulose whose average particle diameter was 290 µm and 16 g of ibuprofen (drug powder) whose average particle diameter was 27 µm were used as powder materials, and these powder materials were subjected to combining treatment for 20 minutes under such condition that the number of revolutions of the cylindrical rotator 3 was 1300 rpm, thereby preparing a drug containing composite particle. Note that, in the present example, there were prepared three kinds of drug containing composite particles by changing the treatment temperature in the combining treatment as 26°C, 66°C, and 84°C. Note that, in the combining treatment, the same powder treatment device as in Example 1 was used.

An ultraviolet absorption spectrum of ibuprofen that had been extracted from thus prepared drug containing composite particle by using methanol was measured, and thus measured ultraviolet absorption spectrum was compared with an ultraviolet absorption spectrum of ibuprofen that had not been combined. A result of the comparison is shown in Fig. 10. Fig. 10 shows a result of measurement performed after filtering methanol extract so as not to receive an influence given by suspension of the methanol extract. Fig. 10 shows that: the ultraviolet absorption spectrum of ibuprofen, that had been combined, was the same as the ultraviolet absorption spectrum of ibuprofen, that had not been combined, regardless of the treatment temperature. That is, after the combining treatment, ibuprofen has the same structure as ibuprofen that has not been treated, i.e., the combining treatment does not chemically change ibuprofen.

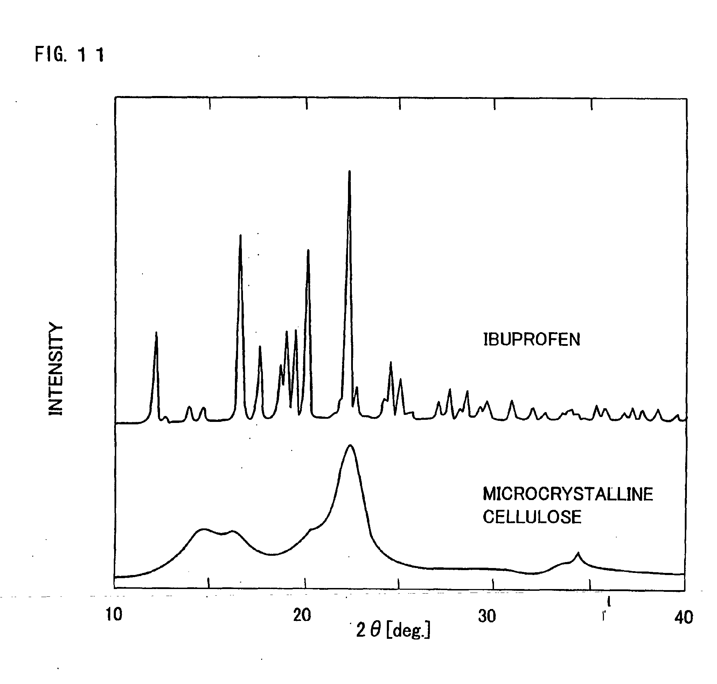

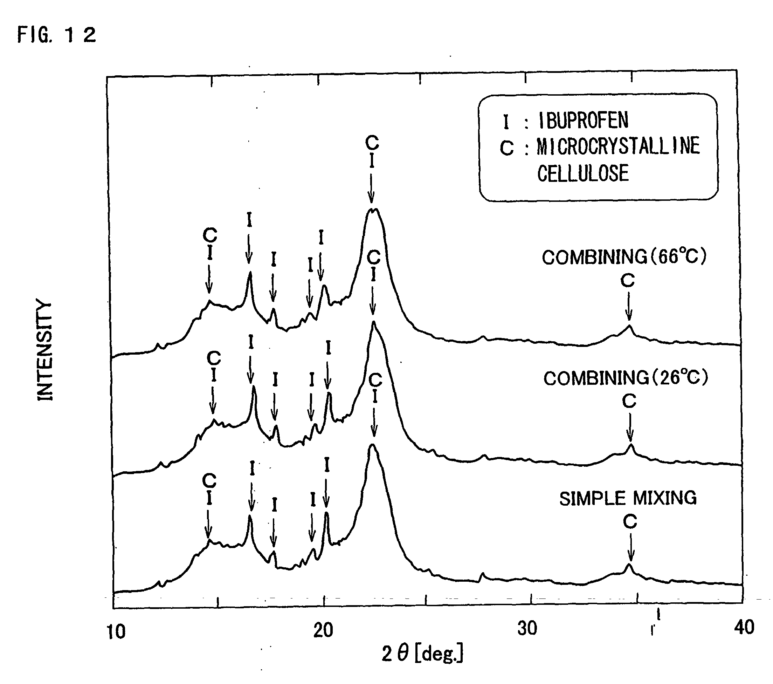

Further, each of Fig. 11 and Fig. 12 shows a result obtained by examining how the combining treatment influences crystalline properties of microcrystalline cellulose and ibuprofen. Fig. 11 shows a measurement result in X-ray analysis performed with respect to microcrystalline cellulose and ibuprofen that had not been combined. Fig. 12 shows a measurement result in X-ray analysis performed with respect to a mixture obtained by simply combining microcrystalline cellulose with ibuprofen, and shows a measurement result in X-ray analysis performed with respect to a drug containing composite particle obtained by the combining treatment.

As shown in Fig. 12, the measurement result in X-ray analysis performed with respect to the drug containing composite particle is the same as the measurement result in X-ray analysis performed with respect to the mixture obtained by simply combining microcrystalline cellulose with ibuprofen, and an angle of each peak caused by the crystalline property of microcrystalline cellulose (the peak is indicated by "C") does not change, and an angle of each peak caused by the crystalline property of ibuprofen (the peak is indicated by "I") does not change. Thus, this shows that the combining treatment does not change the crystalline properties of microcrystalline cellulose and ibuprofen. Note that, the simple combining is treatment in which microcrystalline cellulose and ibuprofen are manually mixed at room temperature.

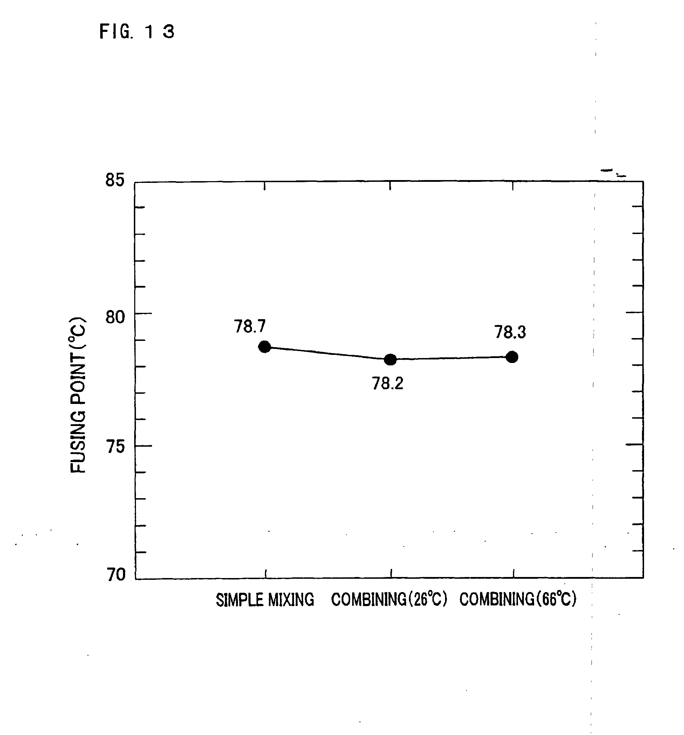

Further, a differential thermal analysis result of Fig. 13 shows that: there is no difference among the resultant obtained by the simple combining, the resultant obtained at the treatment temperature of 26°C, and the resultant obtained at the treatment temperature of 66°C, in terms of the fusing point of ibuprofen. This shows that the combining treatment does not change ibuprofen.

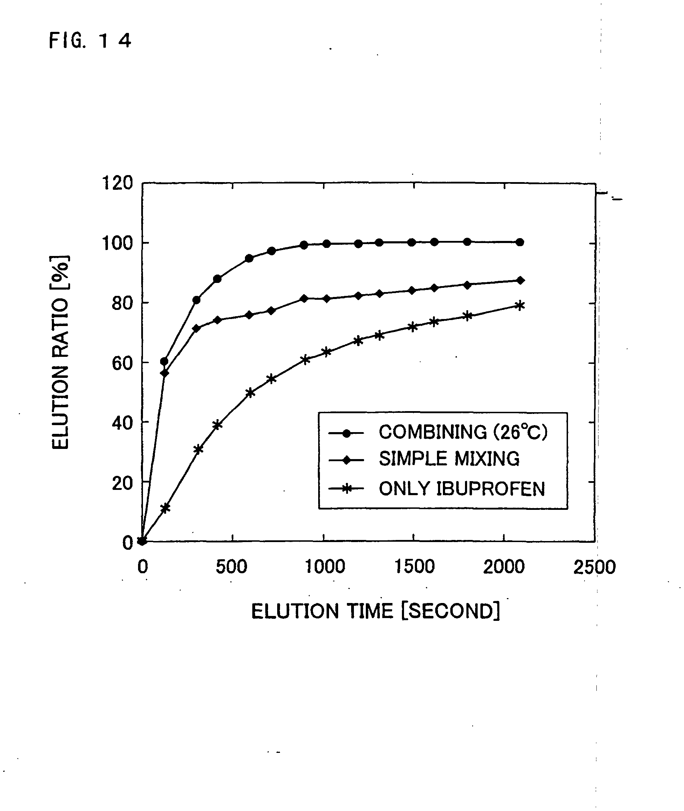

Fig. 14 shows a result obtained by examining how the combining treatment influences an elution ratio of ibuprofen. Measurement of the elution ratio was carried out with respect to (i) the drug containing composite particle obtained by the combining treatment at the treatment temperature of 26°C, (ii) the drug containing composite particle obtained by the simple combining, and (iii) ibuprofen that had not been combined. Here, the elution ratio is a ratio at which ibuprofen dissolves in water.

As shown in Fig. 14, in the drug containing composite particle obtained by the combining treatment, the elution ratio of ibuprofen was high right after commencement of the experiment, and reached substantially 100 % after 1000 seconds had passed as the elution time. This value is much larger than an elution ratio of the drug containing composite particle obtained by the simple mixing and an elution ratio of a particle constituted merely of ibuprofen. This may be based on the following reason: ibuprofen constituting the drug containing a composite particle has a particle diameter which becomes much smaller upon receiving a strong pressure and a strong shearing force in the combining treatment, so that an area contacting water becomes larger. Note that, the elution ratio of ibuprofen is improved by improving a dispersing property by simply combining microcrystalline cellulose with ibuprofen. However, the improvement is limited as shown in Fig. 14.

### [Example 6]

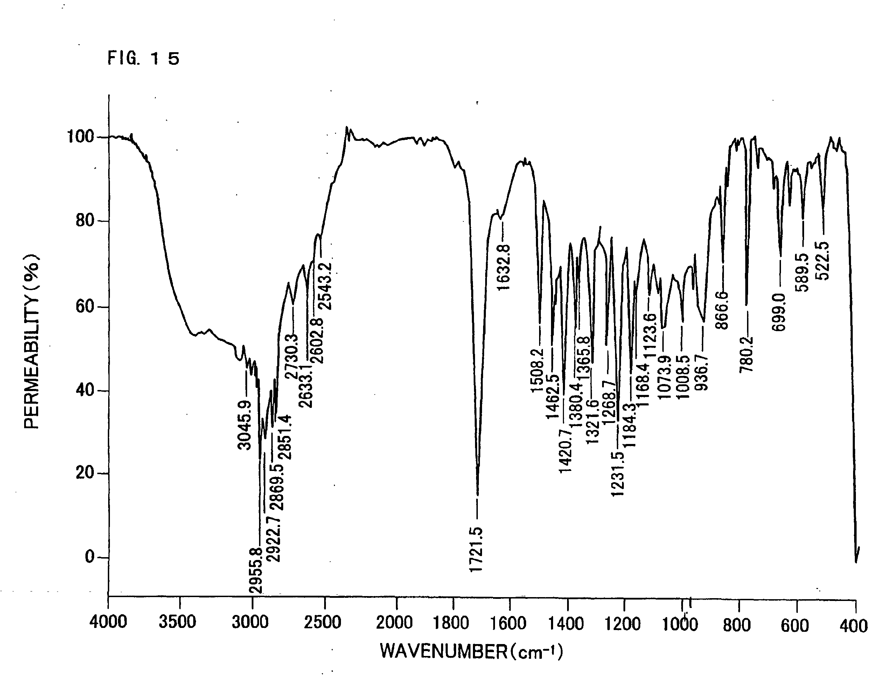

The following description will explain another example of the present invention with reference to Fig. 15 to Fig. 17. A Fourier transform ultraviolet absorption spectrum (FT-IR spectrum) before and after the combining treatment was measured, so as to examine how the combining treatment influences a drug powder. In the present example, the same powder materials and the same powder treatment device as in Example 1 were used under the same condition as in Example 1.

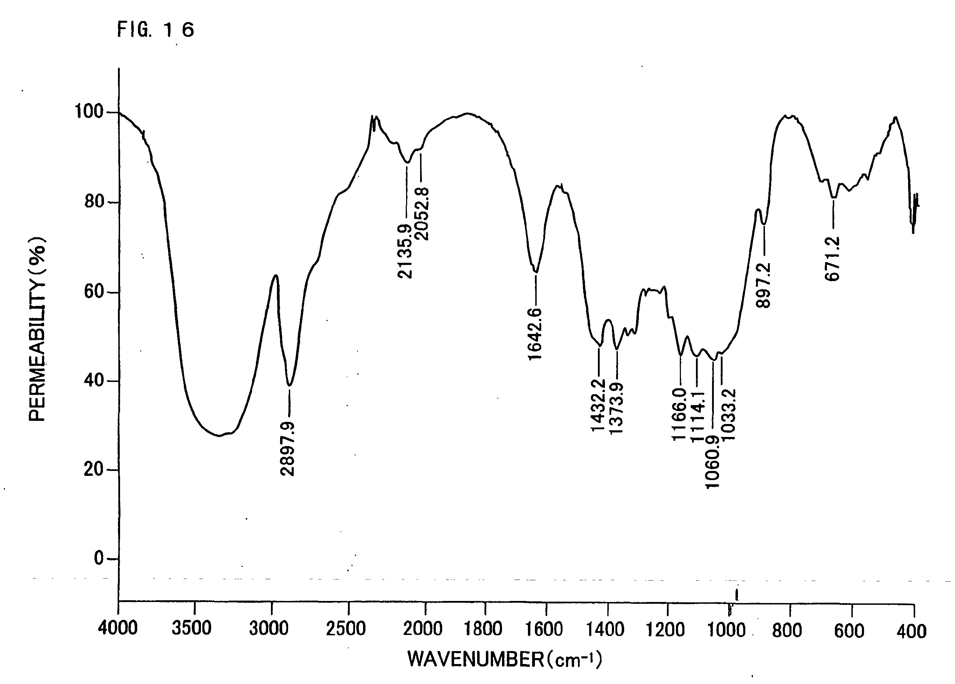

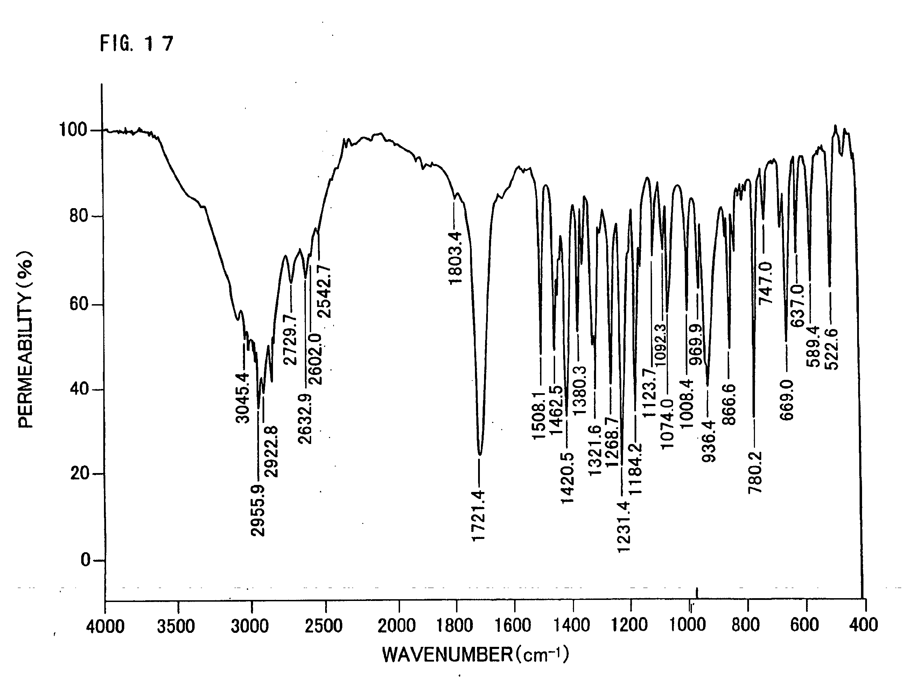

As to a drug containing composite particle obtained by the combining treatment, microcrystalline cellulose, and ibuprofen, each of Fig. 15 to Fig. 17 shows a result obtained by measuring the FT-IR spectrum. These measurement results show that: peaks of the FT-IR spectrum of the drug containing composite particle that are shown in Fig. 15 exist in the FT-IR spectrum of Fig. 16 or Fig. 17.

That is, the FT-IR spectrum of the drug containing composite particle corresponds to a total of the FT-IR spectrum of microcrystalline cellulose constituting the drug containing composite particle and the FT-IR spectrum of ibuprofen constituting the drug containing composite particle. This shows that: microcrystalline cellulose and ibuprofen are not chemically changed by the combining treatment, and no material other than microcrystalline cellulose and ibuprofen is formed. In this manner, also the FT-IR spectrum measurement result shows that the combining treatment does not degenerate the drug.

### [Example 7]

The same operation as in Example 1 was carried out except that: 40 g of microcrystalline cellulose whose average particle diameter was 290 µm and 16 g of acetaminophen (drug powder) whose average particle diameter was 50 µm were used as powder materials, thereby preparing a drug containing composite particle. 4 g of acetaminophen was placed in the powder treatment device in four doses as in Example 1. Note that, acetaminophen is an antipyretic analgesic.

An image of the drug containing composite particle at the time of placing the first 4 g of acetaminophen was taken on the basis of an electron micrograph. As a result, it was confirmed that there was formed the drug containing composite particle in which a surface of microcrystalline cellulose was entirely covered with acetaminophen. Note that, it was visually confirmed that microcrystalline cellulose and acetaminophen were combined with each other at the time of placement of 16 g acetaminophen.

### [Example 8]

12 g of ethenzamide (drug powder) whose average particle diameter was 3 µm and 5.14 g of microparticle titanium oxide whose average particle diameter was 0.015 µm were used as powder materials, so as to carry out combining treatment under such condition that: the treatment temperature was 13.8°C, and the number of revolutions of the cylindrical rotator 3 was 5000 rpm, and the treatment time was 10 minutes, thereby preparing a drug containing composite particle. Note that, the powder treatment device used in the combining treatment was the same powder treatment device as in Example 1. In this manner, ethenzamide and microparticle titanium oxide were combined with each other.

### [Example 9]

20 g of lactose whose average particle diameter was 5 µm and 8 g of ibuprofen (drug powder) whose average particle diameter was 3 µm were used as powder materials, so as to carry out combining treatment under such condition that: the treatment temperature was 20°C, and the number of revolutions of the cylindrical rotator 3 was 4700 rpm, and the treatment time was 20 minutes, thereby preparing a drug containing composite particle. 2 g of ibuprofen was placed in the powder treatment device in four doses. Note that, the powder treatment device used in the combining treatment was the same powder treatment device as in Example 1. In this manner, lactose and ibuprofen were combined with each other.

### [Example 10]

12 g of ibuprofen (drug powder) whose average particle diameter was 3 µm and 5.1 g of microparticle titanium oxide whose average particle diameter was 0.015 µm were used as powder materials, so as to carry out combining treatment under such condition that: the treatment temperature was 20°C, and the number of revolutions of the cylindrical rotator 3 was 5600 rpm, and the treatment time was 10 minutes, thereby preparing a drug containing composite particle. Note that, the powder treatment device used in the combining treatment was the same powder treatment device as in Example 1. In this manner, ibuprofen and microparticle titanium oxide were combined with each other.

As described above, the method of the present invention for producing a drug containing composite particle is a method in which a pressure and a shearing force are exerted to a mixture of two or more kinds of powder materials including a drug powder so as to combine the powder materials.

Therefore, it is possible to combine the drug powder with other powder material without using a binder. Thus, it is possible to produce the drug containing composite particle in a short time without dropping the stability of the drug powder.

Further, the mixture may include a diluting agent powder. Thus, by combining the drug powder and the diluting agent powder with each other, it is possible to produce a drug containing composite particle whose handling property at the time of drug preparation is favorable.

In the method of the present invention for producing the drug containing composite particle, it is preferable that the diluting agent powder is selected from a group of celluloses and starches.

In the method of the present invention for producing the drug containing composite particle, it is preferable that an average particle diameter of the diluting agent powder is not less than one and not more than 10000 times as large as an average particle diameter of the drug powder.

Further, it is preferable that the average particle diameter of the diluting agent powder is not less than 1 µm and not more than 5000 µm, and it is preferable that the average particle diameter of the drug powder is not less than 0.01 µm and not more than 500 µm, and it is preferable that a ratio at which the drug powder is contained in the drug containing composite particle is not less than 0.01 wt % and not more than 90 wt %.

Thus, it is possible to more surely combine the diluting agent powder with the drug powder.

Further, in the method of the present invention for producing the drug containing composite particle, the drug powder may be an antipyretic analgesic or an antiphlogistic.

### [Embodiment 2]

The present embodiment will explain a method for producing a drug containing composite particle including nano-order particles (nano particles) whose average particle diameter is less than 100 nm, and particularly explain a production method of a drug containing composite particle which can be applied to a drug delivery system and the like and enables a composite particle containing drug to be produced.

A nano-order fine particle whose average particle diameter is less than 1000 nm, i.e., less than 1 µm, that is, a nano particle has a much larger specific surface area and a much higher activity than those of conventional fine particles having micron sizes, and its various properties dramatically change, which is highly likely to result in great improvement in product performances in various fields. The nano particle has been so widely applied to production technologies particularly in medicinal drugs and medical products.

Specifically, first, a particle diameter of the nano particle is extremely small. Thus, as to an injectable agent for example, a diameter of a terminal blood capillary is approximately 4 µm, so that it is possible to prevent occurrence of blood clot in intravenous when a solid drug is formed in a nano-particle shape. Further, also in intramuscular injection and hypodermic injection, it is possible to reduce occurrence of inflammation and androblastoma.

Further, a specific surface area of the nano particle is large, which results in increase of surface energy, so that the nano particle shows high reactivity. Therefore, in case of administering a drug via the mouth or the lung, it is possible to improve permeability and an absorbing-portion reaching property of the drug by forming the drug in a nano-particle shape. Further, the nano particle tends to strongly adhere to a surface of a biomembrane, so that an absorbing-portion retention property of the drug is enhanced. As a result, it is possible to enhance absorbability of the drug.

Thus, the nano particle attracts attentions also in terms of development of a Drug Delivery System (hereinafter, referred to as DDS).

However, although the high reactivity of the nano particle is an advantage of the nano particle, the high reactivity destabilizes the nano particle. Therefore, this raises such problem that the stability of the nano particle drug drops before and after administration, or a similar problem.

Then, there was developed such conventional technique that the nano particles are combined with each other by using a chemical method or a physical method so as to improve the stability of the nano particle drug. Specifically, for example, spherical crystallization is adopted so as to combine the drug with a polymer as a composite, thereby forming a polymer nano particle. Note that, as the polymer, it is preferable to use a polymer which is superior in terms of biolysis and biocompatibility.

Incidentally, the high reactivity of the nano particle contributes to the strong surface adherability of the nano particle. However, the strong surface adherability also causes adhesion and cohesion of the nano particles. Therefore, fluidity of the nano particles is extremely low. Furthermore, the nano particle is an ultra-fine particle, so that its volume is larger than that of a conventional micro order fine particle. This raises such problem that a handling property of the nano particle drops.

A combining technique of nano particles such as the aforementioned spherical crystallization sufficiently improve the stability of the nano particle per se, but such technique is not a technique for sufficiently improving the handling property of the stabilized nano particles.

In case of administering drug for example, of course, it is important what influence the drug gives after being administered. However, when the drug is hard to handle before being administered, its quality as a medical product is low no matter how the drug may exhibit a superior drug delivery function. For example, in case of drug which is taken in the body through the lung, a drug powder is filled an supplied in an aspirator. However, when the drug powder is in a nano particle shape, its fluidity is low, so that it is impossible to easily and simply fill and supply the drug powder.

Further, the reactivity of the nano particle causes the drug powder to exhibit superior properties such as a film forming property and a fusing property based on heating and a mechanical stress (pressure, shearing force). Thus, it is possible to develop a new material using these properties (functions). However, when the handling property of the nano particle is low, this influences development of the new material.

It is generally known that granulation is difficult in case of powder having a hydrophobic property and an electrostatic property. The nano particle is an extremely fine particle. Thus, in terms of a design of the particles, it is extremely difficult to granulate such fine particles so that the fine particles are favorably dispersed during and after using the fine particles.

The present invention was devised in terms of the foregoing problem, and its object is to provide a production method of a composite particle whereby it is possible to improve the handling property of the nano particle without losing advantages of the nano particle so that the composite particle can be favorably applied to various medical product fields such as DDS and the like.

The inventors of the present invention earnestly studied the foregoing problems. As a result, they found that: such property that the nano particles tend to clump naturally is used to artificially cause the nano particles to clump so that the nano particles can be dispersed at the time of use, so that it is possible to improve the handling property of the nano particle without losing advantages of the nano particle. Then, they completed the present invention.

The following description will explain one embodiment of the present invention with reference to Fig. 1, Fig. 2, and Fig. 18 to Fig. 21. Note that, the present invention is not limited to this.

A method according to the present embodiment for producing a drug containing composite particle is a method in which: primary particles each of which contains nano particles are formed, and the primary particles are combined with each other so that the primary particles reversibly clump together. As a method for combining the primary particles with each other so that the primary particles reversibly clump together, it is extremely preferable to use a fluid bed dry granulation method or a dry mechanical particle combining method.

The method according to the present invention for producing the drug containing composite particle is favorably used in wide fields such as development of various new materials. Among them, it is possible to favorably use the method particularly in medical products, for example, it is possible to favorably use the method in production of drug powders such as a drug taken in the body through the lung. Note that, in the following description, the drug containing composite particle is referred to as a composite particle as required so as to facilitate the description.

The nano particle of the present invention is a particle whose average particle diameter is less than 1000 nm, that is, a nano-order fine particle. This particle is referred to as a nano sphere. Note that, a particle whose average particle diameter is not less than 1000 nm, that is, not less than 1 µm is referred to as a micron particle.

A material of the nano particle of the present invention is not particularly limited as long as the material can be made into a nano particle shape. In the present embodiment, a nano particle of a drug is described as an example to which the present invention can be applied. However, it is needless to say that a nano particle of other material may be used. That is, in the present embodiment, a drug powder is used as the nano particle. By using the nano particle of a drug, it is possible to favorably use the production method according to the present invention in production of medical products for DDS as described later.

The method of the present invention for producing the nano particle is not particularly limited as long as it is possible to process a target material into particles whose average particle diameter is less than 1000 nm. In the present invention, particularly in case of making a drug into nano particles, it is extremely preferable to use spherical crystallization.

The spherical crystallization is a method in which: a formation/growth process of a crystal at a final stage of compound synthesis is controlled so as to design a spherical crystal particle so that its property is directly controlled, thereby processing the crystal particle. The spherical crystallization is categorized into spherical granulation (SA method) and emulsion solvent dispersion (ESD method) depending on a difference in terms of a formation/clump mechanism of a crystal to be formed.

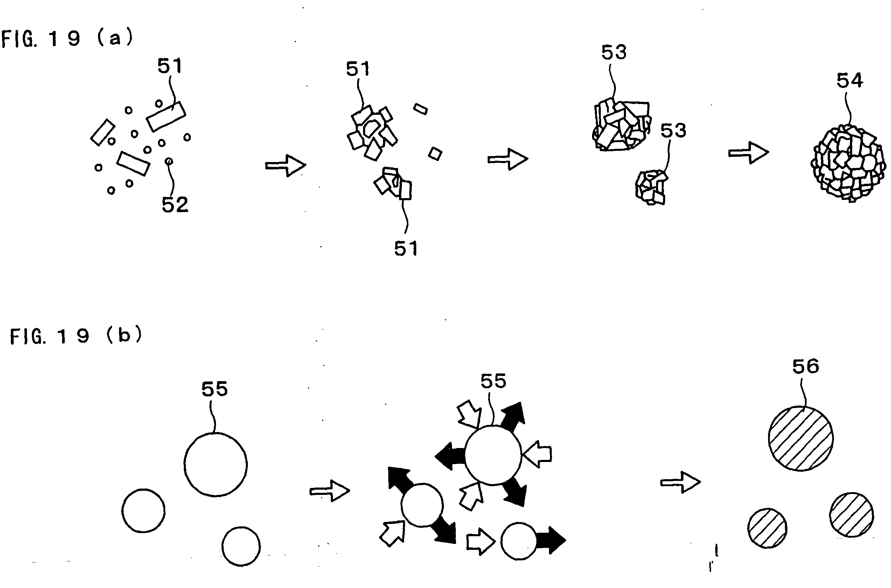

The SA method is a method in which a drug crystal is deposited by using two kinds of solvent so as to form a spherically granulated crystal. Specifically, first, there are prepared (i) a poor solvent in which it is difficult to dissolve a target drug and (ii) a good solvent which enables the drug to be dissolved in a favorable manner and can be mixed and diffused with the poor solvent. Further, a drug solution dissolved in the good solvent is dropped into the poor solvent while being stirred. At this time, by making use of drop in the solubility that is caused by transition from the good solvent to the poor solvent, thermal effect, and the like, a drug crystals 51 are deposited in a system as shown in a leftmost drawing of Fig. 19(a).

Further, when a little amount of liquid (liquid cross-linking agent), having affinity with the drug, which is not mixed with the poor solvent, is added to the system, a liquid cross-linking agent 52 is alienated as shown in the leftmost drawing of Fig. 19(a). Further, the crystals 51 are cross-linked to each other, and a boundary tension and a capillary force cause the crystals 51 to clump in a random manner as shown in the second drawing from the left of Fig. 19(a). Note that, this state is referred to as a funicular state.

When a mechanical shearing force is added to the system in the funicular state, the clumping crystals 51 are consolidated so as to be substantially spherical granulated materials 53 as shown in the third drawing from the left of Fig. 19(a). Note that, this state is referred to as a capillary state. The granulated materials 53 are combined with each other in a random manner, so that a spherically granulated crystal 54 is formed as a final material as shown in the rightmost drawing of Fig. 19(a).

Which kinds of good solvent and poor solvent are to be used and which kind of the liquid cross-linking agent 52 is to be used are determined depending on kinds and the like of the target drug, and are not particularly limited. Further, a condition of the crystallization and an application method of the mechanical shearing force are not particularly limited, and may be determined depending on a kind of the target drug, a particle diameter (nano order in the present invention) of the spherically granulated crystal 54, and the like.

Also the ESD method uses two kinds of solvent, but is different from the SA method in that: emulsion is formed, and mutual diffusion of the good solvent and the poor solvent is utilized so as to crystallize the drug into a spherical shape. Specifically, first, a drug solution dissolved in the good solvent is dropped into the poor solvent while being stirred. At this time, there is affinity between the drug and the good solvent, so that transition from the good solvent to the poor solvent is delayed. As a result, emulsion drops 55 are formed as shown in a left drawing of Fig. 19(b).

Further, as shown in a middle drawing of Fig. 19(b), cooling of the emulsion drops 55 and mutual diffusion of the good solvent and the poor solvent (in this figure, a black arrow indicates diffusion of the good solvent, and a white arrow indicates diffusion of the poor solvent) cause the solubility of the drug to drop in the emulsion drops 55. As shown in a right drawing of Fig. 19(b), drug spherical crystal particles 56 are deposited and grown while retaining shapes of the emulsion drops 55.

As in the SA method, which kinds of the good solvent and the poor solvent are used is determined depending on a kind and the like of the drug, and is not particularly limited. Further, a formation condition of the emulsion and a cooling condition at the time of crystallization are not particularly limited, and may be determined in accordance with a kind of a target drug, a particle diameter (nano order in the present invention) of the spherical crystal particle 55, and the like.

In the spherical crystallization, it is possible to form the nano particles by a physicochemical technique. Moreover, each of thus obtained nano particles has a substantially spherical shape, so that it is possible to easily form even nano particles without taking into consideration such problems that a catalyst or a material compound remains. Further, in using the spherical crystallization for the DDS, there is a case where a drug is modified with biocompatible polymers. In the spherical crystallization, it is possible to form the combined nano particles merely by dissolving the drug and the polymer in the good solvent, so that this is extremely preferable.

A specific kind of a drug formed into a nano particle shape by the spherical crystallization is not particularly limited. Examples of the drug include: an antipyretic analgesic antiphlogistic; a steroid-type antiphlogistic; an antitumor agent; a coronary vasodilator; a peripheral vasodilating drug; an antibiotic; a synthetic antibacterial agent; an antiviral drug; an antispasmodic agent; a cough suppressant; an expectorant; a bronchodilator; a cardiotonic; a diuretic; a muscle relaxant; a brain metabolic stimulant; a minor tranquilizer; a major tranquilizer; a β-blocker; an antiarrhythmic drug; an antipodagric; a blood coagulation inhibitor; a thrombolytic agent; an antihepatism agent; an antiepileptic; an antihistamine; an antiemetic drug; a hypotensive drug; an antihyperlipemia drug; a sympathomimetic drug; an oral antidiabetic medicine; an oral anticancer agent; an alkaloidal narcotic; a vitamin drop; a pollakiuria therapeutic agent; an angiotensin converting enzyme inhibitor; and the like.

In the method according to the present invention for producing a drug containing composite particle, for example, the nano particles formed in the foregoing manner are used to further granulate the nano particles. In the present invention, the nano particle may be used without any modification, but it is more preferable to form secondary particles after forming the primary particles each of which contains the nano particles.

For example, the present invention can be used to produce a dry powder type drug which is taken into the body through the lung. Here, the drug taken in the body through the lung is such that drug powder is temporarily filled and supplied in an aspirator (dry powder spray device), and is sprayed into the mouth.

However, the nano particles are extremely fine particles, so that this causes the handling property such as low fluidity to drop. Thus, when the drug powder is in a nano particle shape, it is impossible to easily and simply fill and supply the drug powder in the aspirator. Furthermore, even when the composite particle is designed so that the composite particle is broken in the mouth so as to directly give off the nano particles, there is a possibility that the composite particles are not broken into the nano particles and a part of the composite particles remains clumped. Therefore, there is a case where it is impossible to deliver the drug to the lung in a favorable manner.

Then, it is so arranged that: the drug powder remains in a micron particle shape while the drug powder passes through the mouth and reaches the lung, and the drug powder becomes in a nano particle shape upon reaching the lung. According to such arrangement, it is possible to favorably disperse even micron particles in the air upon spraying, so that it is possible to improve a ratio at which the drug reaches the lung. Furthermore, the drug powder becomes in a nano particle shape upon reaching the lung, so that it is possible to improve an efficiency at which the drug is absorbed by the lung. In this manner, the arrangement is preferable. Therefore, as the drug taken into the body through the lung, it is possible to use not only a drug favorably administered directly to the lung, e.g., an antiasthmatic agent such as pranlukast hydrate and an antiallergic agent such as a steroid drug, but also a peptide drug such as insulin and calcitonin.

Furthermore, when the nano particles modified with various biocompatible polymers are further combined in accordance with the spherical crystallization, thus obtained composite particle (polymer nano composite particle) can be used as a medical product for the DDS, for example, this arrangement contributes to improvement of the adhesiveness to the lung.

Then, in the present embodiment, it is preferable to secondarily granulate primary particles each of which contains the granulated nano particles. That is, it is preferable that the method of the present invention includes: a nano particle clump formation step of forming primary particles each of which contains nano particles whose average particle diameter is less than 1000 nm; and a combining step of combining the primary particles so that the primary particles are reversibly clumped.

When the foregoing two steps are included, the primary particles each of which contains the nano particles are combined so that they can be dispersed and collected, so that a clumping condition of the primary particles is controlled. Thus, it is possible to design the composite particles so as to be broken into the primary particles each of which contains the nano particles at the time of use thereof.

Thus, the average particle diameter of the composite particle is larger than the nano order before being used, so that its volume is not so large and its fluidity can be made high, and the composite particle can be broken into the primary particles so as to exhibit a function of the nano particle after being used. Therefore, it is possible to improve the handling property of the composite particle without losing advantages of the nano particle.

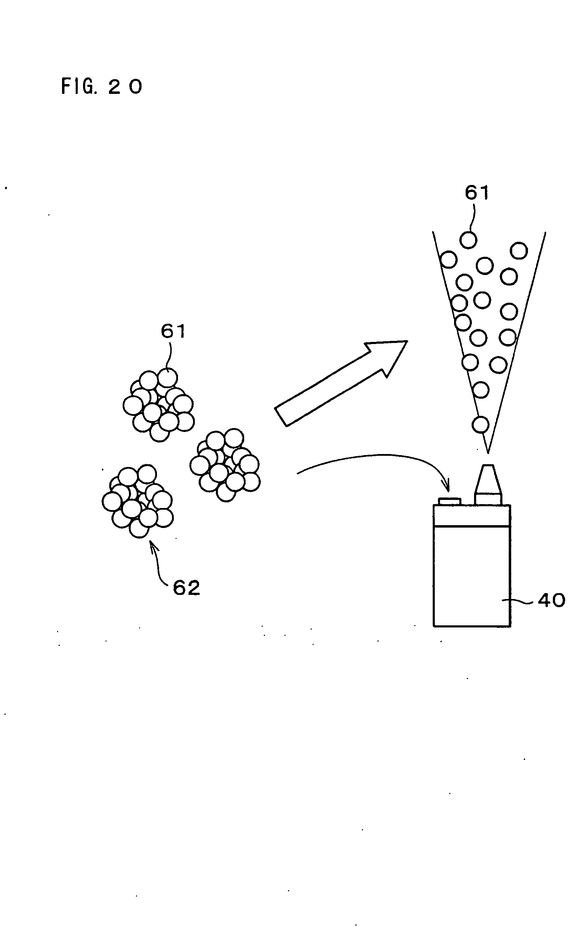

For example, as shown in Fig. 20, in the drug taken into the body through the lung, composite particles 62 are formed by combining the primary particles 61 each of which contains the drug nano particles, and the composite particles 62 are filled and supplied in an aspirator 40. It is possible to form each of the composite particles 62 so that its average particle diameter is approximately dozens µm. Thus, its fluidity and handling property are improved, so that it is possible to easily and simply fill and supply the composite particles 62 in the aspirator 40. Further, the composite particle 62 is easily broken into the primary particles 61 and the primary particles 61 are dispersed, so that each of the fine primary particles 61 passes through the air passage and reaches the lung without fail.

As described above, it is preferable that the primary particle is obtained by clumping a plurality of nano particles. When a condition under which the nano particles are clumped is controlled as required, the composite particle can be broken into the nano particles after being broken into the nano particle clumps. Therefore, it is possible to use the composite particle having a large particle diameter as the nano particle itself at the time of use thereof. As a result, it is possible to improve the handling property while sufficiently making use of advantages of the nano particle.

Further, as described above, particularly in case of forming a drug into a nano particle shape, it is preferable to use the spherical crystallization, so that it is preferable that the method according to the present invention for producing the drug containing composite particle includes a nano particle formation step in which the nano particles are formed in accordance with the spherical crystallization. According to the spherical crystallization, it is possible to carry out the crystallization and the granulation at the same time, so that it is possible not only to form high quality nano particles but also to improve a condition under which the nano particles are designed.

The production method of the present invention including the foregoing steps is described below by explaining production and use of the drug taken into the body through the lung.

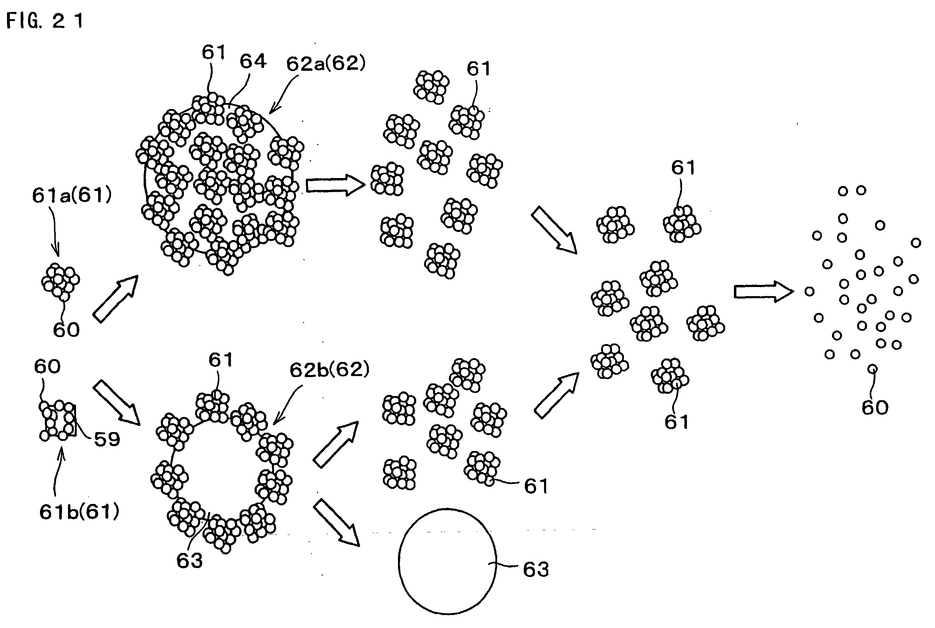

First, in the nano particle formation step, the nano particles are formed in accordance with the spherical crystallization as described above (see Fig. 19(a) and Fig. 19(b)). Next, as shown in Fig. 21, the nano particles are clumped in the primary particle formation step, and the nano particle clump 61 is formed as the primary particle. The primary granulation carried out in the primary particle formation step is not particularly limited, but it is preferable to use a fluid bed dry granulation method described later.

As shown in Fig. 21, the nano particle clump 61 may be a non-carrier-type nano particle clump 61a obtained by clumping only the nano particles 60, or may be a carrier-type nano particle clump 61b obtained by clumping the nano particles 60 via primary carriers 59.

As the primary carrier 59, any material can be used as long as the material has affinity with respect to a living organism or the material can clump the nano particles 60 without giving any bad influence in the living organism. Particularly in case of using the primary carrier 59 in the living organism, it is preferable to use an aqueous compound. Thus, the nano particle clump 61b absorbs moisture (water) regularly retained in the living organism, so that the nano particle clump 61b is easily broken into the nano particles 60.

In the present invention, it is possible to form the carrier-type nano particle clump 61b in accordance with a fluid bed dry granulation method explained in the combining step described later, so that it is possible to favorably use various kinds of binder described later. Of course, the carrier-type nano particle clump 61b may be formed in accordance with other method.

Further, the primary particle is not limited to the nano particle clump 61, but may be a clump constituted of the nano particles 60 and the micron particles. Alternatively, the primary particle may be a drug powder whose surface is modified with the nano particles 60 each of which containins the drug. That is, as the primary particle used in the present invention, any particle may be used as long as the particle contains the nano particles 60 and gives off the nano particles 60 at the stage of final dispersion.

Next, in the combining step, the nano particle clumps 61 are subjected to the secondary granulation, that is, the nano particle clumps 61 are combined with each other, so as to produce a composite particle 62. As the combining step, it is preferable to use the fluid bed dry granulation method or the dry mechanical particle combining method in the present invention. Thus, it is possible to obtain the micron particle (composite particle 62), which is a clump of the nano particles 60, without damaging the structure of the nano particle 60.

Note that, in accordance with the fluid bed dry granulation method, it is possible to obtain a binder-clumping-type composite particle 62a having been subjected to the secondary granulation using a binder 64. In accordance with the dry mechanical particle combining method, it is possible to obtain a carrier-type composite particle 62b having been subjected to the secondary granulation using a carrier particle 63 (different from the primary carrier 59 in the primary granulation).

The composite particle 62 obtained in the combining step is arranged as follows: the primary particles respectively containing the nano particles 60, preferably the nano particle clumps 60, are combined with each other so that they can be dispersed and collected. Thus, in case of using the composite particles 62 in the drug taken into the body through the lung, when the composite particles 62 respectively containing the drug nano particles 60 are sprayed into the mouth after being filled in an aspirator, the composite particles 62 are favorably broken at the time of spray as shown in Fig. 21, so that the nano particle clumps 61 are dispersed and taken into the lung through the mouth as shown in Fig. 20.

More specifically, in case of the binder-clumping-type composite particle 62a obtained in accordance with the fluid bed dry granulation method, the clump obtained by means of the binder 64 is favorably broken, so that the nano particle clumps 61 are given off. In case of the carrier-type composite particle 62b obtained in accordance with the dry mechanical particle combining method, the clump obtained by the adhesion to the surface of the carrier particle 63 is favorably broken at the time of spray, so that the nano particle clumps 61 are given off.

When an average particle diameter of the composite particle 62 is larger than a nano order, for example, when the average particle diameter is dozens to several hundreds µm, the composite particle 62 can be made under such condition that its volume is not so large and its fluidity is favorable. Thus, it is possible to improve its handling property as the drug taken into the body through the lung, at the time of placement into the aspirator. Furthermore, at the time of use thereof, the composite particle 62 is broken into the nano particle clumps 61 (primary particles) each of which has approximately not more than over ten µm, preferably 0.01 µm to 15 µm, and the nano particle clumps 61 are dispersed, and thus dispersed nano particle clumps 61 are favorably absorbed in the lung.

Further, the nano particle clump 61 temporally settles on the lung, but humidity in the lung causes the nano particle clump 61 to absorb water or the primary carrier to be dissolved, so that the nano particle clump 61 is easily broken again into the nano particles 60 and dispersed. Thus, due to the high reactivity of the nano particle 60, there are improved the permeability, the absorbing-portion reaching property, the absorbing-portion retaining property, and the like of the drug in the lung. As a result, it is possible to enhance the absorbability of the drug.

In this manner, when the production method according to the present invention is used, it is possible to improve the handling property of the nano particle 60 without losing its advantages.

Next, the following description will explain the fluid bed dry granulation method and the dry mechanical particle combining method, favorably used in the combining step, which can be used also in the primary particle formation step.

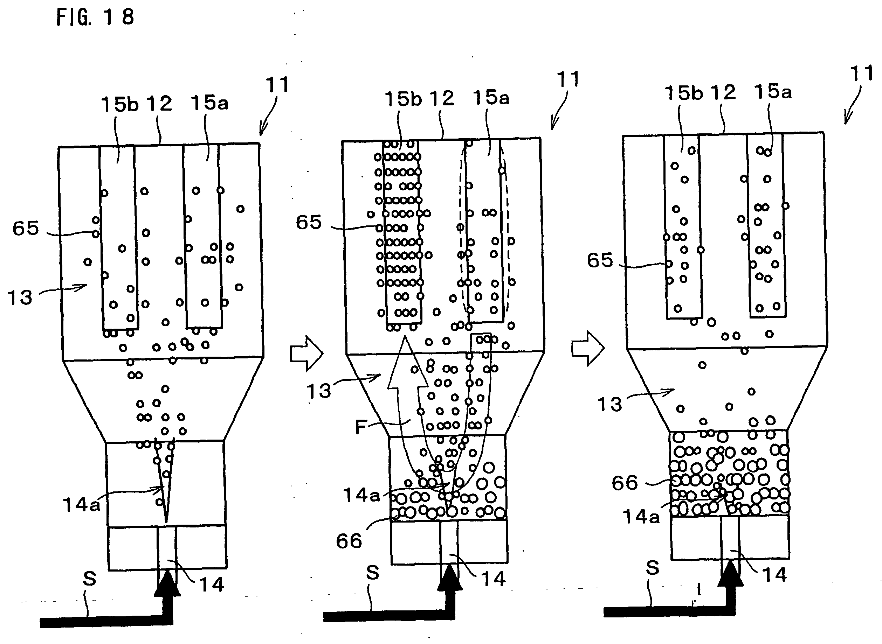

In the fluid bed dry granulation method, it is possible to obtain the binder-clumping-type composite particle 62a specifically by using a fluid bed dry granulation/coating type powder treatment device 11 as shown in Fig. 18.

As shown in Fig. 18, the powder treatment device 11 schematically includes: an upper portion which is a part of a space substantially in a cylindrical shape; a lower portion, having an internal diameter smaller than that of the upper portion, which is a part of the space substantially in the cylindrical shape; a casing 12, connecting the upper portion to the lower portion, which sequentially varies in terms of its internal diameter and has a fluid bed space 13 obtained by integrating the upper portion, the lower portion, and a middle portion whose cross-sectional surface is substantially in a trapezoidal shape; a spray nozzle 14, provided on a lowest portion of the casing 12, which can spray liquid material toward the fluid bed space 13; two bag filters 15a and 15b which are positioned in the upper portion of the fluid bed space 13 so as to protrude downward; a liquid material supplying section (not shown); and an air supplying section (not shown) for supplying a drying/ fluidizing air to the fluid bed space 13.

In the powder treatment device 11, the liquid material is sprayed in the fluid bed space 13 having nothing therein, so that clump granulation and layering granulation of particles are repeated. As a result, it is possible to form granular powder. Therefore, it is possible to simplify device arrangement and a production process, and it is possible to produce high quality powder. Moreover, in case of producing the powder, it is not necessary to put any seed particles therein.

In the present invention, the clump granulation and the layering granulation are carried out, and the liquid material obtained by dispersing and suspending the nano particles or the nano particle clumps is sprayed into the fluid bed space 13, so as to use the nano particles 60 or the nano particle clumps 61 as the seed particles. Further, when the binder 64 is used as the liquid material, it is possible to form the nano particle clumps 61 by clumping the nano particles 60, and it is possible to produce the composite particle 62 according to the present invention by clumping the nano particle clumps 61.

Specifically, first, the liquid material (arrow "S" in Fig. 18), supplied from the liquid material supplying section, into the fluid bed space 13 as shown in a left drawing of Fig. 18. A drop of the sprayed liquid material is so minute that a spray mist diameter of the sprayed liquid material is approximately 10 µm. Thus, the liquid material is solidified in a moment while rising in the fluid bed space 13, and the solidified material becomes fine particles 65. Then, the fine particles 65 are collected in the bag filters 15a and 15b that are positioned on the upper side of the fluid bed space 13.

Here, the fine particles 65 have the following characteristics: in case where the seed particles are the nano particles 60, the binder 64 (that is, the primary carrier 59) adhere to the nano particle 60. Further, in case where the seed particles are the nano particle clumps 61, the binder 64 adheres to the nano particle clump 61.

In each of the bag filters 15a and 15b, the fine particles 65 are intermittently brushed off into the spray zone 24a, positioned under the casing 12, in accordance with pulse jet reverse filtration. The spray zone 14a is an area in which the liquid material is sprayed from the spray nozzle 14. In this area, the liquid material adheres to surfaces of the fine particles 65 and grow while clumping, so as to be powder 66. This process is the clump granulation.

In case where the seed particles are the nano particles 60, the powder 66 become the nano particle clump 61 obtained by clumping the nano particles 60 via the binder 64 (that is, the primary carrier 59). Further, in case where the seed particles are the nano particle clumps 61, the nano particle clumps 61 become the composite particle 62, according to the present invention, which are obtained by secondarily clumping the nano particle clumps 61 via the binder 64.

Further, at the same time of the clump granulation, the liquid material adheres directly to a surface of the powder 66 which has grown larger, and the powder 66 is further dried and solidified. Thus, the powder 66 further grows. This process is the layering granulation.

The clump granulation and the layering granulation continue, so that a fluid bed (arrow "F" in Fig. 18) of the powder 66 is formed in the fluid bed space 13 as shown in Fig. 18. Note that, in Fig. 18, the fluid bed extends from the bag filter 15a to the bag filter 15b, but the present invention is not limited to this.

As to the liquid material supplied thereafter, most of the liquid material adheres to the surface of the powder 66 and extends, and is further deposited, and is dried. That is, the layering granulation is proceeded after the formation of the fluid bed. Therefore, a period in which the layering granulation is performed is adjusted, so that it is possible to control conglobation and weight increase of the powder 66. As a result, it is possible to obtain the powder 66 having an appropriate average particle diameter, that is, it is possible to obtain the nano particle clump 61 or the composite particle 62 as shown in Fig. 18.

Note that, the powder treatment device 11 may be arranged so that it is possible to change an atmosphere in the casing 12, that is, an atmosphere in the fluid bed space 13 depending on the liquid material and a kind of the nano particle 60 or a similar condition. For example, it may be so arranged that various kinds of gas such as inert gas and heating gas is introduced from the air supplying section into the casing 12.

In this manner, in accordance with the fluid bed dry granulation method, the nano particle clump 61 in a fluid state is dried while spraying liquid binder 64, so that the nano particle clump 61 is secondarily clumped via the binder 64. Therefore, it is possible to produce the binder-clumping-type composite particle 62a with high efficiency and high quality. Further, likewise, it is also possible to produce the nano particle clump 61 by using the nano particles 60, obtained in accordance with the spherical crystallization, as the seed particles.

Therefore, in the fluid bed dry granulation method, it is possible to sequentially carry out the formation of the nano particle clump 61 to the composite particle 62 commencing with the formation of the nano particle 60. Thus, it is possible to favorably control a condition under which the nano particles 60 are clumped. As a result, it is possible to further improve the handling property without losing advantages of the nano particles 60.

It is preferable that an average particle diameter of the nano particle clump 61 used in the fluid bed dry granulation method is within a range of from 0.01 µm or more to 500 µm or less. Thus, it is possible to efficiently and surely produce the composite particle 62 obtained by secondarily granulating the nano particle clumps 61. Note that, the present invention can be favorably used to produce the drug taken in the body through the lung. In case of the drug taken in the body through the lung, it is preferable that an average particle diameter of the primary particle, that is, an average particle diameter of the nano particle clump 61 is within a range of from 0.01 µm or more to 15 µm or less.

Further, as the binder 64 used in the fluid bed dry granulation method, it is possible to use aqueous solution of biocompatible polymer. Thus, it is possible to control a secondary granulation state of the composite particle 62 obtained by secondarily granulating the nano particle clumps 61. Particularly, in case of using a drug as the nano particle 60, it is preferable to use the biocompatible polymer.

The biocompatible polymer is selected depending on use of the drug, and is not particularly limited. Examples of the biocompatible polymer include: hydroxypropylmethyl cellulose phthalate; chitosan; lactic acid · glycolic acid copolymer; and the like. Further, not only water, the seed particles (the nano particles 60 or the nano particle clumps 61), the various kinds of biocompatible polymers, but also various kinds of saccharides may be added to the liquid material used in the fluid bed granulation method. Specific examples of the saccharides include oligosaccharides such as lactose, saccharose, mannitol, sorbitol, and the like.

Next, specifically, the powder treatment device 21 shown in Fig. 1 and Fig. 2 is used in the dry mechanical particle combining method. The powder treatment device 21 was explained in Embodiment 1, so that description thereof is omitted in the present embodiment.

In the dry mechanical particle combining method, as the target material 27, a particle mixture obtained by mixing the primary particles, that is, the nano particle clumps 61 with the carrier particle 63 is used, and a pressure and a shearing force are given to the target material 27, so that it is possible to make a plurality of nano particle clumps 61 adhere to the surface of the carrier particle 63. Therefore, also this method enables the composite particle 62 to be produced with high efficiency and high quality, and enables a condition under which the nano particles 60 are clumped to be controlled. As a result, it is possible to further improve the handling property without losing advantages of the nano particles 60.

It is' preferable that: an average particle diameter of the nano particle clump 61 (primary particle) used in the dry mechanical particle combining method is within a range of from 0.01 µm or more to 500 µm or less, and an average particle diameter of the carrier particle 63 is within a range of from 1 µm or more to 500 µm or less. Thus, it is possible to efficiently and surely produce the composite particle 62 constituted of the carrier particle 63 and the nano particle clumps 61 (primary particles). Note that, the present invention can be used to produce the drug taken into the body through the lung. In case of the drug taken into the body through the lung, it is preferable that: an average particle diameter of the primary particle, that is, an average particle diameter of the nano particle clump 63 is within a range of from 0.01 µm or more to 15 µm or less, and an average particle diameter of the carrier particle 63 is within a range of from 10 µm or more to 100 µm or less.

The carrier particle 63 used in the dry mechanical particle combining method is selected depending on use of the drug and the like, and is not particularly limited. Generally, it is preferable to use a biocompatible polymer, particularly, polysaccharide powder or hydrophilic polymer powder as the carrier particle 63. Thus, it is possible to favorably control a combining condition of the composite particle 62 obtained by combining the carrier particle 63 with the nano particle clumps 61 (primary particles), that is, it is possible to favorably control a condition under which the nano particle clumps 61 adhere to the surface of the carrier particle 63.

Specific examples of polysaccharide used as the carrier particle 63 includes: celluloses such as microcrystalline cellulose, methyl cellulose, carmellose sodium, carmellose calcium, and low substituted hydroxypropyl cellulose; and starches such as wheat starch, rice starch, corn starch, potato starch, hydroxypropyl starch, carboxymethyl starch sodium, α-cyclodextrin, and β-cyclodextrin. Further, specific examples of hydrophilic polymer used as the carrier particle 63 include: polymers such as polymethyl methacrylate (PMMA), and the like.

Particularly, in case where a drug is used as the nano particle 60, it is preferable to use microcrystalline cellulose, corn starch, potato starch, and the like, as the polysaccharide powder, and it is preferable to use polymethyl methacrylate as the hydrophilic polymer powder.

It is more preferable that the production method according to the present invention further includes a carrier particle surface modification step in which the surface of the carrier particle 63 is modified in accordance with the fluid bed dry granulation method or the dry mechanical particle combining method before carrying out the combining step. By carrying out the carrier particle surface modification step, it is possible to control a condition under which the primary particles (nano particle clumps 61 and the like) adhere to the surface of the carrier particle 63. Thus, it is possible to control a separability between the primary particle and the carrier particle 63, so that it is possible to favorably break the composite particle 62 into the primary particles and disperse the primary particles.

The surface of the carrier particle 63 is modified so that its shape is spherical from a large view point and an umbonal bump (≤ 0.1 µm) is formed from a small view point, so that it is possible to control adhering/ separating properties between the carrier particle 63 and the primary particle. This is based on the following reason. The carrier particle 63 has more contact points with respect to the primary particles which facilitates the adhesion, but contact areas between the respective particles are smaller, so that van der Waals force decreases. Thus, in case of using this arrangement in the drug taken into the body through the lung, the primary particles are more likely to be separated in an air flow.

Therefore, the surface modification in the carrier particle surface modification step is carried out so that the condition from the large view point and the condition from the small view point are satisfied. Specifically, the modification may be carried out so that the surface of the carrier particle is smoothed in accordance with the fluid bed dry granulation method or the dry mechanical particle combining method, or may be carried out so that the carrier particle 63 and lubricant particles are combined with each other in accordance with the foregoing techniques.

It is possible to easily smooth the surface of the carrier particle 63 by using the powder treatment device 11 or 21 described in the fluid bed dry granulation method or the dry mechanical particle combining method. Likewise, it is also possible to easily combine the carrier particle 63 with the lubricant by using the powder treatment device 11 or 21.

As long as the lubricant particle is made of a material which can control the adhering condition (separating property) between the carrier particle 63 and the primary particle by adhering to the surface of the carrier particle 63, the lubricant particle is not particularly limited. However, it is possible to favorably use various kinds of oligosaccharides generally used as a lubricant and a compound used as a coating agent in a medical drug field.

Specific examples of the lubricant particle include: oligosaccharides such as lactose, saccharose, mannitol, and sorbitol; biocompatible polymers such as hydroxypropyl methyl cellulose phthalate, hydroxypropyl cellulose, carboxymethylethyl cellulose, crystalline cellulose, gum arabic, gelatin, chitosan, polyethyleneglycol, lactic acid and glycolic acid copolymer; inorganic compounds such as calcium carbonate, talc, titania (titanium oxide), and silica (silicon dioxide); interfacial active agents such as sugar ester, and magnesium stearate; and the like.

It is preferable that the lubricant powder are made into a nano particle shape. Thus, it is possible to favorably carry out the combining treatment in accordance with the fluid bed dry granulation method or the dry mechanical particle combining method. For example, it is preferable to use silica as colloidal silica, and it is preferable that the biocompatible polymer is a polymer nano particle (polymer sphere) formed in accordance with the spherical crystallization.

In this manner, the production method of the present invention is a method for secondarily granulating the nano particle so that the nano particle is broken and dispersed at the time of or after use thereof. It is particularly preferable to use the biocompatible polymer in granulation (clumping) of the nano particles or the primary particles for the secondary granulation.

In other words, the present invention can be expressed also as follows: the production method of the present invention is a method for producing a drug containing composite particle which contains a drug and a biocompatible polymer, wherein at least one of the drug and the biocompatible polymer is formed as a nano particle whose average particle diameter is less than 1000 nm, and a mixture containing the nano particle is made into a composite in accordance with a fluid bed dry granulation method or a dry mechanical particle combining method.

Thus, it is possible to obtain a functional micron particle having a nano structure. Thus, for example, it is possible to produce a drug containing composite particle whose nano particles can be sufficiently dispersed at the time of us thereof, and it is possible to produce a drug containing composite particle in which a surface of the drug is modified with biocompatible polymer nano particles so as to improve the drug delivery property. As a result, it is possible to use the present invention in production of medical products whose handling properties are improved without losing advantages of the nano particles.

Typically, the present invention is used in production of medical products, particularly, the present invention is so favorably used in production of the drug taken into the body through the lung. In the present invention, it is possible to favorably control a shape and density of the drug containing composite particle, so that it is possible to design a predetermined aerodynamic diameter in the production of the drug taken into the body through the lung. As a result, it is possible to optimize an inhalation property of the drug powder.

Of course, the present invention is not limited to this. As long as the present invention is favorably used so that the property of the nano particle is sufficiently utilized at the time of use thereof, the present invention is applicable to any use. Thus, it is needless to say that: it is possible to favorably use the present invention in development of new materials based on various kinds of nano technologies other than the production of medical products.

For example, even when the present invention is used exclusively in production and development of medical drugs such as the drug containing composite particle according to the present invention, it is possible to use the antiasthmatic agent and the antiallergic agent not only in the high dispersion type powder inhalation drug (drug taken into the body through the lung) but also in development of DDS drugs taken into the body through the mouth or the lung, instead of injection, by making a diabetic medicine such as insulin into a composite particle. Further, by using adherent nano particles, it is possible to use a borne loss disease medicine such as calcitonin in development of DDS drugs taken into the body through the mouth or the lung.

Further, as to the DDS, by using a hydrophilic polymer such as hydroxypropylmethyl cellulose phthalate, it is possible to use the present invention in development of a drainage pattern enteric coating basis, and it is possible to use the present invention in improving the inhalation properties of (i) a direct compression biodegradable long term information implant basis and (ii) a less absorbable orally administered drug.

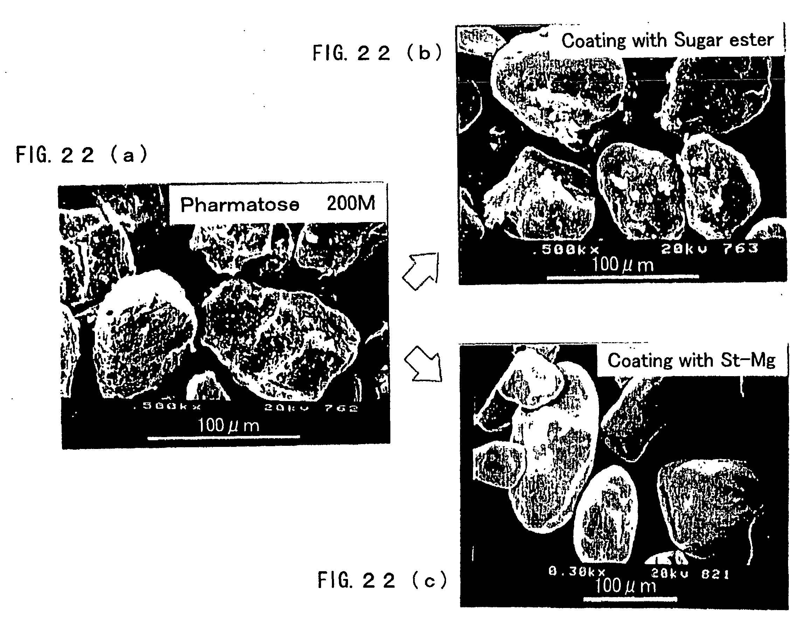

The following Examples and Fig. 22 will further detail the present invention, but the present invention is not limited to this.

### [Example 11]

Pranlukast hydrate particles whose average particle diameter was 1 to 2 µm were used as a model of the primary particle containing nano particles, and 1 kg of the pranlukast hydrate particles were placed in a powder treatment device (see Fig. 18: Aglomaster AGM-2SD made by Hosokawa Micron Corporation) based on the fluid bed dry granulation method, and the pranlukast hydrate particles were fluidized while operating a spiral flow pulse jet dispersion device. At this time, a fluidization (dry) air quantity was 0.8 m³/min and inlet air temperature was 80°C. Further, hydroxypropyl cellulose (HPC) whose temperature was 5°C or less was sprayed and supplied (approximately 300 g of HPC in case of 5 % aqueous solution) from a spray nozzle in a bottom of the powder treatment device, thereby preparing granules (composite particles) whose average particle diameter was 30 to 40 µm as a binder.

Thus obtained granules were filled and supplied in an aspirator made by Unisia Jecs Corporation, and then were sprayed. A condition of the granules that had not been sprayed and a condition of the granules that had been sprayed were observed. It was simple and easy to fill and supply the granules, and each of the granules was favorably broken into primary particles.

Thus obtained granules were formed after the dispersion and clumping steps carried out in accordance with the fluid bed dry granulation method, so that the granules were extremely superior in terms of a dispersing/breaking property. Therefore, the granule functions as a granule superior in terms of the fluidity in being supplied and filled in an aspirator, and the granule is favorably broken and thus broken particles favorably disperse due to the dispersion effect in the inhalation. Thus, each of fine primary particles thereof can pass through the respiratory passage to reach the lung.

### [Example 12]

As a carrier particle, a lactose whose average particle diameter was 40 µm was used as a carrier particle. 50 g of the lactose was placed in a powder treatment device (see Fig. 5 and Fig. 6: AM-MINI (mechanofusion mini), made by Hosokawa Micron Corporation, which has a treatment capacity of 100 ml/batch, a casing internal diameter Φ of 80 mm, a cool water jacket, and a rotor maximum rotation number of 3500 rpm), and was subjected to the treatment for 15 minutes with the rotor rotation number of 2500 rpm. Note that, water was circulated in the cool water jacket so that powder temperature was 40°C or less. This is because: when the lactose is not cooled with water, the powder temperature rises with time, so that the lactose fuses and adheres to the powder treatment device over 80°C.

Under such condition, a pressure and a shearing force of the rotor caused a surface of the carrier particle to be smoothed, thereby carrying out surface modification. The carrier particle of the lactose whose surface had been treated was not made amorphous.

Thereafter, pranlukast hydrate particles (model of the primary particles) whose average particle diameter was 1 to 2 µm were made to adhere to the surface of the carrier particle by using a nauta mixer (inverted-cone-type mixer), thereby forming granules. The granules having been filled and supplied in an aspirator were sprayed. Then, a condition of the granules that had not been sprayed and a condition of the granules hat been sprayed were observed. It was simple and easy to fill and supply the granules, and each of the granules was favorably broken into primary particles.

### [Example 13]

As in Example 12, lactose whose average particle diameter was 40 µm was used as the carrier particle. 40 g of the lactose and 5 g of sugar ester which functions as the lubricant were placed in the AM-MINI so that these materials were subjected to the treatment for 20 minutes with the rotor rotation number of 3500 rpm. Note that, as in Example 12, the cool water jacket was used, and the powder temperature was set to be 40°C or less. The lactose was prevented from being amorphous.

Thereafter, granules were formed in the same manner as in Example 12, and were filled and supplied in an aspirator. Thereafter, the granules were sprayed. Then, a condition of the granules that had not been sprayed and a condition of the granules that had been sprayed were observed. It was simple and easy to fill and supply the granules, and each of the granules was favorably broken into primary particles. Further, Fig. 22 shows a result obtained by observing the surface of the carrier particle by means of an electronic microscope before and after carrying out the surface modification with the sugar ester (a left drawing is a 500-power image obtained before the surface modification, and an upper right drawing is a 300-power image obtained after the surface modification).

### [Example 14]

The same operation was carried out as in Example 13 except for that 5 g of magnesium stearate (St-Mg) was used as the lubricant, thereby preparing granules. Thus obtained granules were filled and supplied in an aspirator, and then were sprayed. Then, a condition of the granules that had not been sprayed and a condition of the granules that had been sprayed were observed. It was simple and easy to fill and supply the granules, and each of the granules was favorably broken into primary particles. Further, Fig. 22 shows a result obtained by observing the surface of the carrier particle by means of an electronic microscope before and after carrying out the surface modification with the St-Mg (a left drawing is a 500-power image obtained before the surface modification, and a lower right drawing is a 300-power image obtained after the surface modification).

As apparent from Fig. 22, the carrier particle (crushed lactose) whose surface had been modified with the lubricant had a smoother and polished surface than that of the carrier particle whose surface had not been modified. Thereafter, the adhering and separating property between the primary particle and the carrier particle was remarkably improved (controlled).

### [Example 15]

The same operation was carried out as in Example 13 except that 5 g of colloidal silica was used as the lubricant, thereby preparing granules. Thus obtained granules were filled and supplied in an aspirator, and were sprayed. Then, a condition of the granules that had not been sprayed and a condition of the granules that had been sprayed were observed. It was simple and easy to fill and supply the granules, and each of the granules was favorably broken into primary particles.

As apparent from a result of Fig. 22, the surface of the lactose was modified in accordance with the dry mechanical particle combining method, so that the sugar ester (upper right drawing of Fig. 22) and the magnesium stearate (lower right drawing of Fig. 22) were evenly bonded to the surface of the lactose in a dispersion manner. Thus, compared with an untreated material (left drawing of Fig. 22), the fluidity of the granules (composite particles) and the dispersing property which caused the particle to be broken into the primary particles were remarkably improved. As a result, the adhering and separating property between the primary particle and the carrier was remarkably improved (controlled).

### [Example 16]

The same operation was carried out except that starch was used as the carrier particle, thereby preparing granules. Thus obtained granules were filled and supplied in an aspirator, and were sprayed. Then, a condition of the granules that had not been sprayed and a condition of the granules that had been sprayed were observed. It was simple and easy to fill and supply the granules, and each of the granules was favorably broken into primary particles.

### [Example 17]

A lactose solution prepared as 20 % aqueous solution was sprayed, granulated, and dried with Aglomaster AGM-2SD, thereby obtaining spherical fine particles (carrier particles) whose average particle diameter was 35 µm. During the operation, the spiral flow pulse jet dispersion device was operated as required (sprayed the fine particles for 0.3 seconds and stopped spraying the fine particles for 5 seconds with an air pressure of 5kgf/cm²), and thus obtained fine particles were spirally dispersed along a fluid bed inner wall surface (the fine particles were rolled on the wall surface), thereby promoting conglobation and smoothing of the carrier particle. Note that, a fluidization (dry) air quantity was 0.8 m³/min, and inlet air temperature was 80°C, and exhaust air temperature was 45°C, and a lactose solution supplying rate was 10 g/min, and an amount of the lactose solution supplied was 1000 g.

Thereafter, as in Example 12, granules were formed, and thus formed granules were filled and supplied in an aspirator, and were sprayed. Then, a condition of the granules that had not been sprayed and a condition of the granules that had been sprayed were observed. It was simple and easy to fill and supply the granules, and each of the granules was favorably broken into primary particles.

### [Example 18]

The carrier particles obtained in Example 17 were used. Subsequently, magnesium stearate (alcohol: 200 g of 5 % dispersion liquid) was supplied to the AGM-2SD, so as to coat a surface of the fine particle prepared in advance.

Thereafter, as in Example 17, granules were formed, and thus formed granules were filled and supplied in an aspirator, and were sprayed. Then, a condition of the granules that had not been sprayed and a condition of the granules that had been sprayed were observed. It was simple and easy to fill and supply the granules, and each of the granules was favorably broken into primary particles.

Particularly, when the magnesium stearate was bonded to the carrier particle surface that had been smoothed, the resultant was extremely superior in 'terms of the dispersing property which caused the particle to be broken into the primary particles.

As described above, the method according to the present invention for producing a drug containing composite particle includes: a primary particle formation step of forming primary particles each of which contains a nano particle having an average particle diameter of 1000 nm or less; and a combining step of combining the primary particles so that the primary particles are reversibly clumped, wherein it is preferable to carry out the combining step in accordance with a fluid bed dry granulation method or a dry mechanical particle combining method, and a biocompatible polymer is used to carry out the combining step.

Further, it is preferable that the primary particle is a nano particle clump obtained by clumping a plurality of nano particles, and it is preferable that the production method includes a carrier particle surface modification step, carried out before the combining step, in which a surface of the carrier particle is modified in accordance with the fluid bed granulation method or the dry mechanical particle combining method in case of using the carrier particle in a combining treatment.

According to the foregoing method, the primary particles each of which contains the nano particles are combined with each other so that the primary particles can be dispersed and collected, so that a condition under which the primary particles are clumped is controlled. Therefore, it is possible to design the drug containing composite particle so that the drug containing composite particle is broken into the primary particles each of which contains the nano particles at the time of use thereof.

Thus, an average particle diameter of the drug containing composite particle is larger than a nano order before begin used, so that its volume is not required to be large and its fluidity can be made high, and the drug containing composite particle can be broken into the primary particle so as to exhibit a function of the nano particle in (after) being used. Therefore, it is possible to improve the handling property of the drug containing composite particle without losing advantages of the nano particle.

Use of the method according to the present invention for producing a drug containing composite particle is not particularly limited. The production method is used in case where properties of the nano particles can be sufficiently utilized. For example, the production method is extremely favorably used in production of a powder drug which is delivered to the lung and is taken into the body through the lung. In the present invention, it is possible to favorably control a shape and density of the drug containing composite particle, so that it is possible to design a predetermined aerodynamic diameter in the production of the drug taken into the body through the lung. As a result, it is possible to optimize an inhalation property of the drug powder.

In this manner, the present invention can be applied to development of future-generation DDS corresponding to an aging society by means of an environment-friendly mechanical process. Therefore, it is possible to establish technologies applicable to medical drugs and medical products based on a nano technology.

### [Embodiment 3]

The present embodiment will explain a method for producing a drug containing composite particle which contains nano-order particles (nano particles) whose average particle diameter is less than 100 nm, particularly, a method for producing a drug containing composite particle which can be favorably used in a drug delivery system and the like by modifying a surface of a drug powder.

Medical drugs are required to have various properties such as tractability (handling property) in production, masking of bitterness, solubility control, DDS (Drug Delivery System), and the like. Thus, a plurality of materials have been combined as a composite in order to give necessary properties.

Examples of the composite constituted of the plural materials include: a composite constituted of a diluting agent and drug; a composite obtained by coating a surface of a drug with a lubricant or a coating agent; and the like. Here, the diluting agent improves the handling property of the drug and makes it easier to compress the drug into a medical drug. The lubricant lubricates a surface of the drug. The coating agent covers the surface of the drug so as to mask bitterness of the drug for example.

Among them, a technique for coating a surface of the drug with various kinds of surface modification agent such as a lubricant and a coating agent attracts attentions also in terms of development of a Drug Delivery System (hereinafter, referred to as DDS).

For example, as a method for administering a drug, administration through the lung attracts attentions recently. The lung has a wide absorption area substantially equal with the digestive tube, and an epithelium of the air cell is thin, and a vascular system is developed under the epithelium. Thus, the lung is advantageous in terms of permeation and absorption of a material, and its enzymatic activity is relatively low, and a drug can be taken directly into a general circulation system. Particularly, the lung is very effective as a general administration route.

As a method for delivering a drug into the lung, there was used such a conventional method that: a mixture obtained by dissolving or suspending a drug in a volatile spray agent such as chlorofluorocarbon was aerosolized, and thus aerosolized mixture was inhaled. However, use of the chlorofluorocarbon is restricted, so that it is developed such a recent method that: a drug is made into a dry powder shape, and the dry-powder drug is inhaled. According to such technique, a drug is made into a powder shape so as to have a micron order, and a surface of the drug powder is modified with a lubricant such as colloidal silica, thereby improving its fluidity. Thus, it is possible to improve the fluidity and a dispersing property of the drug powder, so that it is possible to promote absorption of the drug.

However, according to the conventional technique in which the surface of the drug is modified, it is possible to modify the surface to some extent, but this technique raises such problem that: the surface modification is not sufficiently controlled and the productivity of the drug powder is not sufficient.

Specifically, the lubricant such as colloidal silica is in a nano-order fine particle shape, that is, in a nano particle shape, so that it is not easy to modify the surface of the powder with the lubricant. Therefore, it is difficult to appropriately modify the surface of the powder depending on a kind and use of the drug. Further, it is often that a rotor type powder combining device is used, but such device cannot sufficiently control the surface modification and is not suitable for mass production of the drug powder whose surface has been modified.

The present invention was devised from the foregoing view point, and its object is to provide a production method of a drug containing composite particle by which: it is possible to favorably modify a surface of a drug powder with nano particles, and it is possible to favorably control the surface modification, and it is possible to improve productivity of the drug powder whose surface has been modified.

The inventors of the present invention diligently studied the foregoing problems. As a result, they found that: by combining nano particles with a drug particle in accordance with a fluid bed dry granulation method or a dry mechanical particle combining method, it is possible to favorably control the surface modification and it is also possible to improve the productivity of the drug powder whose surface has been modified. As a result, they completed the present invention.

The following description will explain one embodiment of the present invention with reference to Fig. 1 to Fig. 2. Note that, the present invention is not limited to this.

A method according to the present embodiment for producing a drug containing composite particle is a method in which a mixture containing (i) nano particles whose average particle diameter is less than 1000 nm and (ii) a drug powder whose average particle diameter is larger than the average particle diameter of the nano particles is formed as a composite, so as to modify a surface of the drug powder.

The method according to the present invention for producing the drug containing composite particle is favorably used in wide fields such as development of various medical drugs and medical products. Among them, it is possible to favorably use the method particularly in production of a drug powder such as drug taken into the body through the lung and it is possible to use the drug containing powder component for the DDS.

The nano particle of the present invention is a particle whose average particle diameter is less than 1000 nm, that is, a nano-order fine particle, and is expressed also as a nano sphere. Note that, a particle whose average particle diameter is 1000 nm or more, that is, a particle whose average particle diameter is 1 µm or more is expressed as a micron particle.

A material of the nano particle of the present invention is not particularly limited as long as the material can be made into a nano particle shape. However, it is preferable to use a material which does not have any bad influence to a living body in case where the material is used in combination with a drug. Of course, the nano particle itself may be a drug.

A specific kind of the drug used in the present invention is not particularly limited. Examples of the drug include: an antipyretic analgesic antiphlogistic; a steroid-type antiphlogistic; an antitumor agent; a coronary vasodilator; a peripheral vasodilating drug; an antibiotic; a synthetic antibacterial agent; an antiviral drug; an antispasmodic agent; a cough suppressant; an expectorant; a bronchodilator; a cardiotonic; a diuretic; a muscle relaxant; a brain metabolic stimulant; a minor tranquilizer; a major tranquilizer; a β-blocker; an antiarrhythmic drug; an antipodagric; a blood coagulation inhibitor; a thrombolytic agent; an antihepatism agent; an antiepileptic; an antihistamine; an antiemetic drug; a hypotensive drug; an antihyperlipemia drug; a sympathomimetic drug; an oral antidiabetic medicine; an oral anticancer agent; an alkaloidal narcotic; a vitamin drop; a pollakiuria therapeutic agent; an angiotensin converting enzyme inhibitor; and the like.

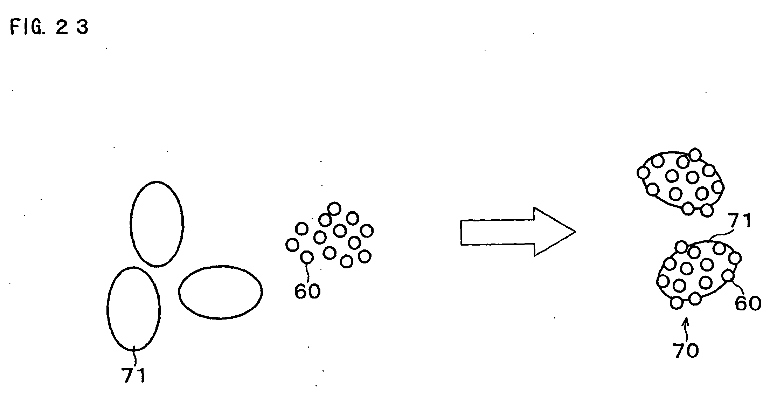

In the method according to the present invention for producing the drug containing composite particle, for example, nano particles 60, each of which is constituted of biocompatible materials, and a drug powder (for example, a micron particle) 71 are combined with each other as shown in Fig. 23, thereby producing a drug containing composite particle 70. Thus, it is possible to further improve the absorbability of the drug.

For example, the present invention can be used in production of a powder type drug taken into the body through the lung. Specifically, the drug taken in the body through the lung is such that a drug powder is filled and supplied in an aspirator (dry powder spraying device), and is sprayed into a mouth. The sprayed drug powder enters the body through the mouth and reaches the lung.

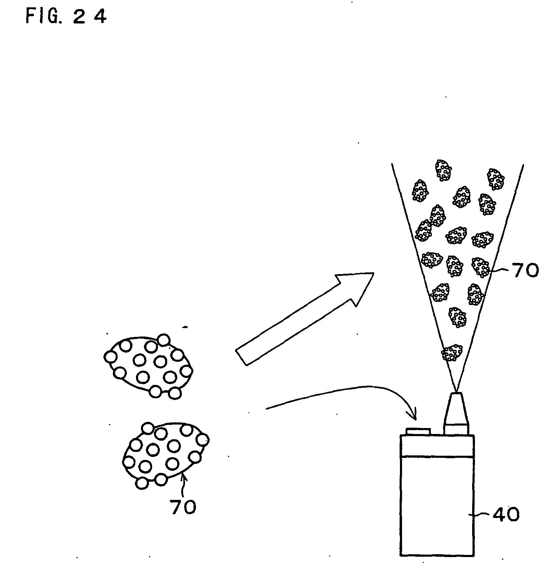

Here, it is general that the drug powder is in a micron particle shape. The drug powder which is in the micron particle shape is made into a drug containing composite particle 70 whose surface has been modified with nano particles. Then, thus obtained drug containing composite particles 70 are filled and supplied in an aspirator 40 as shown in Fig. 24, and are sprayed into the mouth. Thus, after being delivered from the mouth to the lung, it is possible to favorably make the drug containing composite particle 70 settle on the lung and it is possible to further improve the absorbability of the drug. Therefore, when the present invention is used, as the drug taken into the body through the lung, it is possible to use a peptide drug, such as insulin and calcitonin, that has been conventionally regarded as being difficult to use as the drug taken into the body through the lung.

Further, the micron particle is a fine particle though it is not as fine as the nano particle, so that the fine micro particle has a low handling property such as low fluidity. Thus, it is impossible to easily and simply fill and supply the drug powder in the aspirator. Moreover, when the composite particle is broken in the mouth and directly gives off the nano particles, the nano particles are clumped again or a similar reaction occurs, so that it is impossible to favorably disperse the nano particles in the air. By using the production method of the present invention, it is possible to improve the fluidity of the drug powder in the aspirator, and it is possible to improve a dispersing property in the air.

In the present embodiment, the nano particles are formed by using a biocompatible polymer, and the nano particles and the drug powder are combined with each other, so that the surface of the drug is modified. That is, in the present invention, the production method includes a combining step in which a mixture containing (i) nano particles whose average particle diameter is less than 1000 nm and (ii) a drug powder whose average particle diameter is larger than the average particle diameter of the nano particles is formed into a composite in accordance with a fluid bed dry granulation method or a dry mechanical particle combining method.

According to the method, the nano particle and the drug powder are combined with each other in accordance with the fluid bed dry granulation method or the dry mechanical particle combining method, so that it is possible to effectively use the nano particles as powdery surface modification agent. As a result, compared with conventional surface modification using the micro particles, it is possible to favorably modify the surface of the drug power, and it is possible to favorably control a level of the surface modification. Further, the fluid bed dry granulation method and the dry mechanical particle combining method are suitable for mass treatment. Thus, it is possible to further improve the productivity of the drug powder whose surface has been modified.

As the nano particles used in the present invention, any material can be used as long as the material can modify a surface of a target drug and can improve the absorbability and the fixability in the living body. However, it is preferable to use various kinds of lubricant powders.

As the lubricant, it is possible to favorably use various kinds of oligosaccharides, used as general lubricants, and compounds, used as coating agents in a medical field, for example.

More specifically, examples of the lubricant include: oligosaccharides such as lactose, saccharose, mannitol, and sorbitol; biocompatible polymers such as hydroxypropyl methyl cellulose phthalate, hydroxypropyl cellulose, carboxymethylethyl cellulose, crystalline cellulose, gum arabic, gelatin, chitosan, polyethyleneglycol, lactic acid and glycolic acid copolymer; inorganic compounds such as calcium carbonate, talc, titania (titanium oxide), and silica (silicon dioxide); interfacial active agents such as sugar ester, and magnesium stearate; and the like.

Each of the compounds is made into a nano particle shape. In case of an inorganic compound for example, the inorganic compound is in a colloidal shape. Specifically, it is possible to favorably use commercial colloidal silica in case of silica.

While, it is preferable to make the biocompatible polymer into a nano particle shape in accordance with spherical crystallization. That is, it is preferable that the production method of the present invention includes a nano particle formation step in which the nano particles used as the surface modification agent are formed in accordance with the spherical crystallization. According to the spherical crystallization, it is possible to carry out the crystallization and the granulation at the same time, so that it is possible to not only to form high quality nano particles but also to improve a condition under which the nano particles are designed.

The spherical crystallization is a method in which: a formation/growth process of a crystal at a final stage of compound synthesis is controlled so as to design a spherical crystal particle so that its property is directly controlled, thereby processing the crystal particle. The spherical crystallization is categorized into spherical granulation (SA method) and emulsion solvent dispersion (ESD method) depending on a difference in terms of a formation/clump mechanism of a crystal to be formed.

The SA method is a method in which a drug crystal is deposited by using two kinds of solvent so as to form a spherically granulated crystal. Specifically, first, there are prepared (i) a poor solvent in which it is difficult to dissolve a target drug and (ii) a good solvent which enables the drug to be dissolved in a favorable manner and can be mixed and diffused with the poor solvent. Further, a drug solution dissolved in the good solvent is dropped into the poor solvent while being stirred. At this time, by making use of drop in the solubility that is caused by transition from the good solvent to the poor solvent, thermal effect, and the like, crystals of the target material are deposited in a system.

Further, when a little amount of liquid (liquid cross-linking agent), having affinity with the drug, which is not mixed with the poor solvent, is added to the system, a liquid cross-linking agent is alienated. Further, the crystals are cross-linked to each other, and a boundary tension and a capillary force cause the crystals to clump in a random manner (funicular state). When a mechanical shearing force is added to the system in the funicular state, the clumping crystals are consolidated so as to be substantially spherically granulated materials (capillary state). The granulated materials are combined with each other in a random manner, so that a spherically granulated crystal is formed as a final material.

Which kinds of good solvent and poor solvent are to be used and which kind of the liquid cross-linking agent 52 is to be used are determined depending on kinds and the like of the target drug, and are not particularly limited. Further, a condition of the crystallization and an application method of the mechanical shearing force are not particularly limited, and may be determined depending on a kind of the target drug, a particle diameter (nano order in the present invention) of the spherically granulated crystal, and the like.

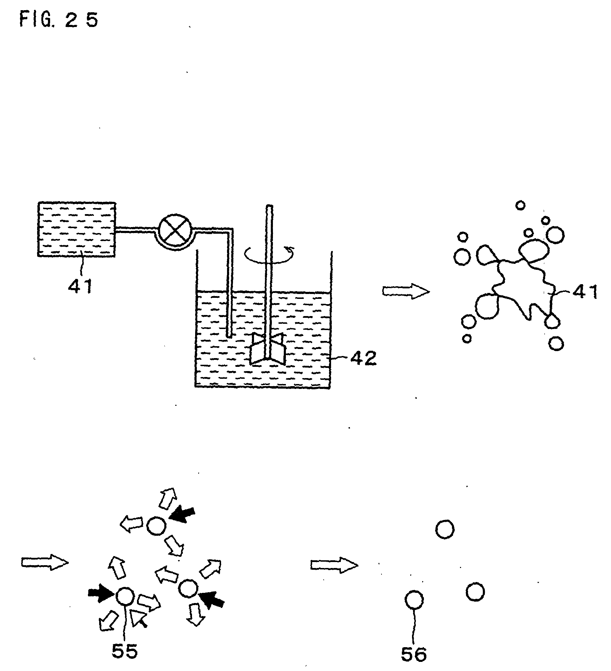

In the present invention, particularly in case of making the biocompatible polymer into a nano particle shape, it is more preferable to use the ESD method. Also the ESD method uses two kinds of solvent, but is different from the SA method in that: emulsion is formed, and mutual diffusion of the good solvent and the poor solvent is utilized so as to crystallize the drug into a spherical shape. Specifically, first, as shown in an upper left drawing of Fig. 25, a drug solution 41 dissolved in the good solvent is dropped into the poor solvent 42 while being stirred. At this time, as shown in an upper right drawing of Fig. 25, the polymer solution 41 quickly diffuses in the poor solvent. Then, as shown in a lower left drawing of Fig. 25, emulsion drops 55 are formed in accordance with self emulsion (Marangoni effect).

Further, as shown in a lower drawing of Fig. 25, cooling of the emulsion drops 55 and mutual diffusion of the good solvent and the poor solvent (in this figure, a white arrow indicates diffusion of the good solvent, and a black arrow indicates diffusion of the poor solvent) cause the solubility of the drug to drop in the emulsion drops 55. As shown in a lower right drawing of Fig. 25, drug spherical crystal particles 56 are deposited and grown while retaining shapes of the emulsion drops 55.

As in the SA method, which kinds of good solvent and poor solvent are used is determined depending on a kind and the like of the target polymer, and is not particularly limited. Further, a formation condition of the emulsion and a cooling condition at the time of crystallization are not particularly limited, and may be determined in accordance with a kind of a target polymer drug, a particle diameter (nano order in the present invention) of the spherical crystal particle 55, and the like.

In the spherical crystallization, it is possible to form the nano particles by a physicochemical technique. Moreover, each of thus obtained nano particles has a substantially spherical shape, so that it is possible to easily form even nano particles without taking into consideration such problems that a catalyst or a material compound remains. Further, in the spherical crystallization, it is possible to form the combined nano particles merely by dissolving the drug and the polymer in the good solvent, so that this is extremely preferable.

In the present embodiment, as a polymer nano particle (polymer nano sphere) obtained by the spherical crystallization, it is particularly preferable to use a particle constituted of lactic acid glycolic acid copolymer or hydroxymethyl cellulose phthalate. Thus, it is possible not only to favorably modify the surface of the drug powder with polymer but also to favorably control a level of the surface modification performed with polymer. Further, the polymer nano particle may include not only the biocompatible polymer but also various kinds of drugs. Thus, it is possible to obtain such an advantage that the surface nano-particulate drug and the internal drug particle can be administered at the same time.

Next, the following description will explain a flow of the production method of the present invention which includes the foregoing steps by giving an example where the nano particle (polymer nano sphere) containing the biocompatible polymer is used.

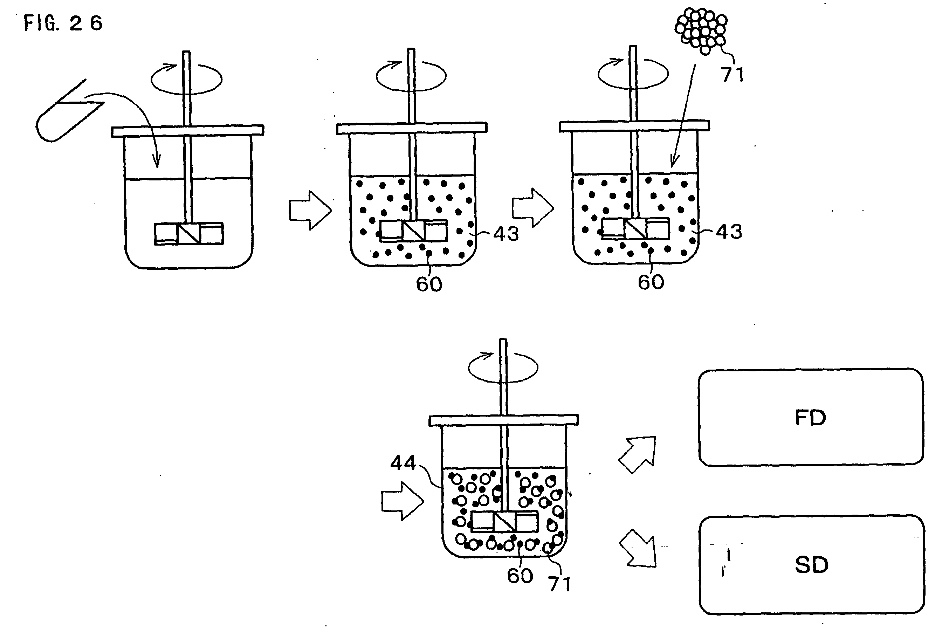

First, as shown in an upper left drawing of Fig. 26, the nano particle is formed in the nano particle formation step as described above (see Fig. 25). Thus, as shown in an upper center drawing of Fig. 26, there is prepared a dispersion liquid 43 in which the nano particles 60 are dispersed. Further, as shown in an upper right drawing of Fig. 26, a drug powder 71 is added to the nano particle dispersion liquid 43, and thus obtained resultant is sufficiently stirred. Thus, as shown in a lower left drawing of Fig. 26, there is prepared a nano particle/drug dispersion liquid 44 including the drug powder 71 and the nano particles 60.

In the present invention, a composite particle 70 is produced by using the nano particles/drug dispersion liquid 44. However, in the production of the composite particle 70, the combining operation is performed in accordance with the fluid bed dry granulation method or the dry mechanical particle combining method as shown in a lower left drawing of Fig. 26 (combining step). Note that, in Fig. 26, the fluid bed dry granulation method is referred to as SD, and the dry mechanical particle combining method is referred to as FD.

Next, the fluid bed dry granulation method and the dry mechanical particle combining method are respectively detailed as follows.

In the fluid bed dry granulation method, the fluid bed dry granulation/coating type powder treatment device 11 as shown in Fig. 18 is used. The power treatment device 18 was explained in Embodiment 2, so that description thereof is omitted.

In the present embodiment, the clump granulation and the layering granulation that were described in Embodiment 2 are carried out, and the nano particle/drug dispersion liquid 44 is sprayed into the fluid bed space 13 as a liquid material, and the nano particles 60 and the drug powder 71 are used as seed particles.

It is preferable that an average particle diameter of the drug powder 71 used in the fluid bed dry granulation method is within a range of from 0.01 µm or more and 500 µm or less. Thus, it is possible to efficiently and surely produce the drug containing composite particle 70 according to the present invention.

Next, in the dry mechanical particle combining method, specifically, the powder treatment device 21 as shown in Fig. 1 and Fig. 2 is used. The powder treatment device 21 was described in Embodiment 1, so that description thereof is omitted.

In the dry mechanical particle combining method, a particle mixture obtained by drying the nano particle/drug dispersion liquid 44 is used as the target material 27, and a pressure and a shearing force are exerted to the target material 27, thereby making a plurality of nano particles 60 adhere to the surface of the drug particle 71. Therefore, it is possible to produce the drug containing composite particle 70 with high efficiency and high quality also by using the foregoing method.

Also an average particle diameter of the drug powder 71 used in the dry mechanical particle combining method is within a range of from 0.01 µm or more and 500 µm or less as in the fluid bed dry granulation method. Thus, it is possible to efficiently and surely produce the drug containing composite particle 70 according to the present invention.

Here, in the production method according to the present invention, it may be so arranged that: the drug powder whose particle surfaces have been modified are clumped so as to carry out the second granulation, thereby producing a drug containing composite particle. That is, the production method of the present invention may include a second granulation step in which the drug powder whose particle surfaces have been modified are subjected to the second granulation.

An arrangement of the second granulation is not particularly limited. It may be so arranged that the drug powder is clumped via a binder so as to produce a binder-clumping-type drug powder clump (drug containing composite particle), or it may be so arranged that the drug powder is clumped via a carrier particle so as to produce a carrier-type drug powder clump (drug containing composite particle).

In the second granulation step, as in the combining step, it is so preferable to use the fluid bed dry granulation method or the dry mechanical particle combining method in the present invention. Thus, it is possible to obtain the drug containing composite particle, that has been subjected to the secondary granulation, by clumping the drug powder, whose particle surfaces have been modified, without damaging the structure of the drug powder. Note that, in the fluid bed dry granulation method, the binder-clumping-type drug powder clump is obtained. In the dry mechanical particle combining method, the carrier-type drug powder clump is obtained.

The drug powder clump (drug containing composite particle) obtained in the secondary granulation is such that: particles of the drug powder, each of which is a primary particle containing nano particles and has a surface having been modified, are combined with each other so that the drug powder particles can be dispersed and collected. Thus, in case of using the drug powder clump in the drug taken into the body through the lung, when the drug powder clump is put in an aspirator and is then sprayed into the mouth, the drug powder clump is favorably broken at the time of spray, so that particles of the drug powder whose particle surfaces have been modified are dispersed. As a result, the drug powder is inhaled from the mouth to the lung. Thus, it is possible to favorably use the drug containing composite particle as an inhalation drug.

Note that, specific examples of polysaccharides used as the carrier particle include: celluloses such as microcrystalline cellulose, methyl cellulose, carmellose sodium, carmellose calcium, low substituted hydroxypropyl cellulose, and the like; starches such as wheat starch, rice starch, corn starch, potato starch, hydroxypropyl starch, carboxymethyl starch sodium, α-cyclodextrin, β-cyclodextrin, and the like. Further, examples of hydrophilic polymers used as the carrier particle include polymers such as polymethyl methacrylate (PMMA), and the like. Among them, it is preferable to use the polysaccharides such as microcrystalline cellulose, corn starch, potato starch, and it is preferable to use the hydrophilic polymer such as polymethyl methacrylate.

In this manner, the present invention is used typically in production of medical products, particularly, the present invention is so favorably used in production of a high dispersion type powder inhalation drug such as an antiasthmatic agent and an antiallergic agent (drug taken into the body through the lung). It is needless to say that: the present invention is not limited to them, and it is possible to favorably use the present invention in development of other medical drugs and medical products.

For example, a diabetic medicine such as insulin is made into a composite particle, so that it is possible to use the present invention in development of DDS drugs taken into the body through the mouth or the lung instead of drug injection. Further, by using adherent nano particles, it is possible to use a borne loss disease medicine such as calcitonin in development of DDS drugs taken into the body through the mouth or the lung.

Further, as to the DDS, by using a hydrophilic polymer such as hydroxypropylmethyl cellulose phthalate, it is possible to use the present invention in development of a drainage pattern enteric coating basis, and it is possible to use the present invention in improving the inhalation properties of (i) a direct compression biodegradable long term information implant basis and (ii) a less absorbable orally administered drug such as a peptide drug and the like.

The following Examples will further detail the present invention, but the present invention is not limited to this.

### [Example 19]

35 g of pranlukast hydrate of an antiasthmatic agent (average particle diameter was 3 µm) which was inferior in terms of wettability, fluidity, dispersing property, and the like, and 3.5 g of polymer nano sphere HP-55 (hydroxypropylmethyl cellulose phthalate: average particle diameter was 52 nm), dried by the powder treatment device (see Fig. 18: Aglomaster AGM-2SD made by Hosokawa Micron Corporation) based on the fluid bed dry granulation method, were placed in the powder treatment device (see Fig. 1 and Fig. 7: AM-MINI (mechanofusion mini), made by Hosokawa Micron Corporation, which has a treatment capacity of 100 ml/batch, a casing internal diameter Φ of 80 mm, a cool water jacket, and a rotor maximum rotation number of 3500 rpm), and were subjected to the treatment for 15 minutes with the rotor rotation number of 1500 rpm. Note that, water was circulated in the cool water jacket so that powder temperature was 30°C or less. This is because: when the mixture is not cooled with water, the powder temperature rises with time, so that the mixture fuses and adheres to the powder treatment device over 80°C.

Thus obtained drug containing composite particles were filled and supplied in an aspirator made by Unisia Jecs Corporation, and then were sprayed. Thereafter, a dispersion condition in the air was confirmed. Further, the wettability was confirmed by dispersing thus obtained drug containing composite particles in water. It was simple and easy to fill and supply the drug containing composite particles, and the dispersion condition in the air and the wettability were favorable.

### [Example 20]

The same operation as Example 1 was carried out except that 3.5 g of colloidal silica was used as the nano particle and the rotor rotation number was 2250 rpm. Thus obtained drug containing composite particle was filled and supplied in an aspirator, and was then sprayed. Thereafter, a dispersion condition in the air was confirmed. Further, the wettability was confirmed by dispersing thus obtained drug containing composite particles in water. It was simple and easy to fill and supply the drug containing composite particles, and the dispersion condition in the air and the wettability were favorable.

In this manner, when the method according to the present invention for producing the drug containing composite particle was used, the surface of the pranlukast hydrate was favorably modified, and its dispersion property and wettability were greatly improved.

### [Example 21]

Ethanol/water = 8/2 solution of HP-55 (hydroxypropylmethyl cellulose phthalate) was dropped into water while being stirred so as to form polymer nano spheres, and pranlukast hydrate whose average particle diameter was 2 µm was added to the system right after the formation, thereby preparing nano particle/drug dispersion liquid. The nano particle/drug dispersion liquid was treated by the powder treatment device AGM-2SD based on the fluid bed dry granulation, thereby obtaining a drug containing composite particle whose surface had been modified with HP-55.

Thus obtained drug containing composite particle was filled and supplied in an aspirator, and was then sprayed. Thereafter, a dispersion condition in the air was confirmed. Further, the wettability was confirmed by dispersing thus obtained drug containing composite particles in water. It was simple and easy to fill and supply the drug containing composite particles, and the dispersion condition in the air and the wettability were favorable.

### [Example 22]

The present example is different from Example 3 in that: carrier particles (whose average particle diameter ranged from 30 to 50 µm for example) were placed in the powder treatment device AGM-2SD based on the fluid bed dry granulation method, and the nano particle/drug dispersion liquid was sprayed and supplied to a surface of each carrier particle, thereby obtaining drug containing composite particles. Thus obtained drug containing composite particles were drug powder, clumping on the surface of the carrier particle, whose particle surfaces had been modified.

Thus obtained drug containing composite particle was filled and supplied in an aspirator, and was then sprayed. Thereafter, a dispersion condition in the air was confirmed. Further, the wettability was confirmed by dispersing thus obtained drug containing composite particles in water. It was simple and easy to fill and supply the drug containing composite particles, and the dispersion condition in the air and the wettability were favorable. Thus, it is possible to use the drug containing composite particle as an inhalation drug.

As described above, the method according to the present invention for producing the drug containing composite particle is a method in which: a mixture containing nano particles whose average particle diameter is less than 1000 nm and a drug powder whose average particle diameter is larger than the average particle diameter of the nano particles is made into a composite particle in accordance with a fluid bed dry granulation method or a dry mechanical particle combining method, so as to modify a surface of the drug powder.

In the foregoing method, it is preferable to use the lubricant as the nano particles. As the lubricant, a colloidal inorganic compound powder or an surfactant powder may be used, or polymer nano particles obtained by the spherical crystallization may be used. Further, it is preferable that an average particle diameter of the drug powder is within a range of from 0.01 µm or more to 500 µm or less.

According to the foregoing method, the nano particles and the drug powder are combined with each other by using the fluid bed dry granulation method or the dry mechanical particle combining method. Thus, it is possible to effectively use the nano particles as a surface modification agent for the drug powder. As a result, it is possible to favorably modify the surface of the drug powder, and it is possible to favorably control a level of the surface modification.

Further, the fluid bed dry granulation method and the dry mechanical particle combining method are suitable for mass treatment. Thus, it is possible to further improve the productivity of the drug powder whose surface has been modified.

In this manner, the present invention can be applied to development of future-generation DDS corresponding to an aging society by means of an environment-friendly mechanical process. Therefore, it is possible to establish technologies applicable to medical drugs and medical products based on a nano technology.

The invention being thus described, it will be obvious that the same way may be varied in many ways. Such variations are not to be regarded as a departure from the spirit and scope of the invention, and all such modifications as would be obvious to one skilled in the art are intended to be included within the scope of the following claims.

### INDUSTRIAL APPLICABILITY

As described above, the method according to the present invention for producing the drug containing composite particle is useful in modifying the surface of the powder material so as to give a higher function to the composite particle containing a drug, and is useful particularly as a method, being applicable particularly to a drug delivery system and the like, by which the drug containing composite particle containing nano-order particles is produced.

## Claims

1. A method for producing a drug containing composite particle, being **characterized by** comprising the step of giving a pressure and a shearing force to a mixture, constituted of two or more kinds of powder materials including a drug powder, so as to combine the powder materials with each other.

2. The method as set forth in claim 1, wherein the mixture includes a diluting agent powder.

3. The method as set forth in claim 2, wherein the diluting agent powder is selected from a group of celluloses and starches.

4. The method as set forth in claim 2 or 3, wherein an average particle diameter of the diluting agent powder is not less than one and not more than 10000 times as large as an average particle diameter of the drug powder.

5. The method as set forth in claim 2 or 3, wherein an average particle diameter of the diluting agent powder is 1 µm or more and 5000 µm or less.

6. The method as set forth in claim 1 or 2, wherein an average particle diameter of the drug powder is 0.01 µm or more and 500 µm or less.

7. The method as set forth in claim 1 or 2, wherein a ratio at which the drug powder is contained in the drug containing composite particle is 0.01 wt % or more and 90 wt % or less.

8. The method as set forth in claim 1 or 2, wherein the drug powder is an antipyretic analgesic or an antiphlogistic.

9. A method for producing a drug containing composite particle containing a drug and a biocompatible polymer, being **characterized by** comprising the steps of: making at least one of the drug and the biocompatible polymer into a nano particle whose average particle diameter is less than 1000 nm; and making a mixture containing the nano particle into a composite particle in accordance with a fluid bed dry granulation method or a dry mechanical particle combining method, so as to form a polymer nano composite particle.

10. A method for producing a drug containing composite particle, being **characterized by** comprising: a primary particle formation step of forming primary particles each of which includes nano particles whose average particle diameter is less than 1000 nm; and a combining step of combining the primary particles with each other so that the primary particles are reversibly collected, wherein a drug powder is used as the nano particles or the primary particles.

11. The method as set forth in claim 10, wherein each of the primary particles is a nano particle clump obtained by clumping a plurality of the nano particles.

12. The method as set forth in claim 10 or 11, further comprising a nano particle formation step of forming the nano particles in accordance with spherical crystallization.

13. The method as set forth in claim 10 or 11, wherein, in the combining step, the primary particles are subjected to secondary granulation in accordance with a fluid bed dry granulation method.

14. The method as set forth in claim 13, wherein an average particle diameter of the primary particles is within a range of from 0.01 µm or more to 500 µm or less.

15. The method as set forth in claim 13 or 14, wherein a binder is used to combine the primary particles with each other in the fluid bed dry granulation method.

16. The method as set forth in claim 15, wherein the binder is an aqueous solution of a biocompatible polymer.

17. The method as set forth in claim 10 or 11, wherein, in the combining step, the primary particles are made to adhere to a surface of each of carrier particles, which are larger than the primary particles in terms of an external diameter, in accordance with a dry mechanical particle combining method.

18. The method as set forth in claim 17, wherein: an average particle diameter of the primary particles is within a range of from 0.01 µm or more to 500 µm or less, and an average particle diameter of the carrier particles is within a range of from 1 µm or more to 500 µm or less.

19. The method as set forth in claim 17 or 18, wherein a polysaccharide powder or a hydrophilic polymer powder is used as the carrier particle.

20. The method as set forth in claim 17 or 18, further comprising a carrier particle surface modification step of modifying the surface of the carrier particle, in accordance with a fluid bed dry granulation method or the dry mechanical particle combining method, before carrying out the combining step.

21. The method as set forth in claim 20, wherein, in the carrier particle surface modification step, the surface of the carrier particle is smoothed in accordance with the fluid bed dry granulation method or the dry mechanical particle combining method, or the carrier particle is combined with lubricant particles, so as to modify the surface of the carrier particle.

22. The method as set forth in any one of claims 9, 10, and 11, being used to produce a powdery drug which is delivered to a lung and is absorbed through the lung.

23. A method for producing a drug containing composite particle, being **characterized by** comprising the step of making a mixture, containing nano particles whose average particle diameter is less than 1000 nm and a drug powder whose average particle diameter is larger than the average particle diameter of the nano particles, into a composite particle in accordance with a fluid bed dry granulation method or a dry mechanical particle combining method, so as to modify a surface of the drug powder.

24. The method as set forth in claim 23, wherein a lubricant powder is used as the nano particles.

25. The method as set forth in claim 24, wherein a colloidal inorganic compound powder or a surfactant powder is used as the lubricant powder.

26. The method as set forth in claim 25, wherein the colloidal inorganic compound powder is colloidal silica.

27. The method as set forth in claim 25, wherein the surfactant powder is magnesium stearate or sugar ester.

28. The method as set forth in claim 23, wherein a polymer nano particle obtained in accordance with spherical crystallization is used as the lubricant powder.

29. The method as set forth in claim 28, wherein the polymer nano particle is constituted of a lactic acid glycolic acid copolymer or hydroxymethyl cellulose phthalate.

30. The method as set forth in claim 23 or 24, wherein the average particle diameter of the drug powder is within a range of from 0.01 µm or more and 500 µm or less.

31. The method as set forth in claim 23 or 24, being used to produce a powdery drug which is delivered to a lung and is absorbed through the lung.
